# EUROPEAN PATENT SPECIFICATION

(11) **EP 2 222 232 B1**
(45) Date of publication and mention of the grant of the patent: **12.12.2018**
(21) Application number: 08838963.0
(22) Date of filing: 17.10.2008
(51) Int. Cl.: A61B 17/04

(54) **Devices for locking and/or cutting tethers**
Vorrichtungen zum Abschluss und/oder Schneiden von Spannseilen
Dispositifs pour terminaison et/ oú coupe des tendeurs

(30) Priority: 19.10.2007 US 190036; 28.08.2008 US 92703; 10.10.2008 US 104681
(43) Date of publication of application: 01.09.2010
(73) Proprietor: Ancora Heart, Inc., Santa Clara, CA 95054 (US)
(72) Inventor: SERINA, Eugene, Union City CA 94587 (US); CALHOUN, Tenny, C., Sunnyvale, CA 94087 (US); MEIER, Stephen, deceased (US); FABRO, Mariel, San Jose,CA 95131 (US); MIRCHANDANI, Tiffany, Huynh, San Jose CA 95112 (US); TO, John, Newark CA 94560 (US); TANG, Brian, Fremont CA 94539 (US)
(74) Representative: Cooley (UK) LLP
(86) International application number: PCT/US2008/080381
(87) International publication number: WO 2009/052438

(56) References cited:
- WO-A-2005/110241
- WO-A-2007/056502
- WO-A-2008/112740
- US-A- 5 133 723
- US-A1- 2007 005 081
- US-A1- 2007 106 310

## Description

### TECHNICAL FIELD

The devices and methods described herein relate generally to termination of tethers that have been deployed to a target site in a body of a subject. More specifically, the devices and methods described herein relate to locking and/or cutting such tethers after they have been deployed to the target site.

### BACKGROUND

Many different types of medical procedures involve the use of tethers. For example, tethers may be used to tighten or compress tissue (e.g., by bringing two pieces or sections of tissue together). The tissue may be, for example, soft tissue, such as muscle tissue or fat tissue. As an example, in some procedures, anchors coupled to a tether are embedded in tissue, and the tether is then pulled upon to provide a cinching effect that tightens or compresses the tissue via the anchors. Examples of devices and methods for such procedures applied to heart valve repair are described, for example, in U.S. Patent Application Publication Nos. US 2006/0190030 A1, US 2006/0122633 A1, and US 2008/0172035 A1.

Some methods of tissue tightening include threading a tether through two pieces of tissue, applying tension to the tether, and tying off or knotting the tether to maintain the tension. Extra tether may then be cut and removed. However, the manipulation required when knotting, tying, and/or cutting a tether can be difficult (e.g., because of restricted space). Moreover, certain methods may not adequately maintain tension in a tether. Additionally, some methods of knotting, tying, and/or cutting a tether may be unduly complicated and/or time-consuming.

Accordingly, it would be desirable to provide methods and devices for effectively locking and/or cutting a tether to help maintain tension in the tether. It would further be desirable for such methods and devices to be relatively easy and/or efficient to use.

WO 2005/110241 A1 discloses a device for cutting a surgical suture. A tubular member has a longitudinal axis and a tubular wall with a pair of apertures extending therethrough, the apertures being sized and arranged to permit a surgical suture to pass into the tubular member through one of the pair of apertures and out of the tubular member through the other. A cutting member is received within the tubular member. The device also includes means for causing longitudinal movement of the cutting member and tubular member with respect to one another in a direction to cause the cutting member to pass at least one of the pair of apertures to sever the suture passing therethrough.

WO 2007/056502 A1 discloses a termination device for locking an implantable and cinchable tether. The device comprises: an elongate body; and a locking feature releasably attached to the distal end of the elongate body, the locking feature configured to couple to the tether; the locking feature having an unsecured state, wherein the tether may move with respect to the locking feature, and a secured state, wherein the tether is secured by the locking feature.

US 2007/005081 A1 discloses a tissue fastening device including a suture clip holder and a suture cutter. A suture clip is releasably secured to the suture clip holder, and a suture line or lines pass through the clip and adjacent the suture cutter. A first movement of a control handle causes the suture clip to be released from the suture clip holder, and a second movement of the control handle causes the suture cutter to cut the suture line or lines. The first movement is different from the second movement. The first movement can be rotational, with the second movement being longitudinal. The device includes a main body, an inner body, and an outer body.

### BRIEF SUMMARY

According to aspects of the present invention there is provided the device of claim 1 or claim 12. Additional aspects of the invention are set out in the dependent claims.

Described here are devices and methods for locking and/or cutting tethers, such as tethers that have been used to tighten or compress tissue (e.g., by pulling two or more pieces or sections of the tissue together).

Certain variations of devices described here may be used to cut a tether. For example, some variations of devices described here comprise a cutter disposed within a lumen of an elongated member, such as a catheter. In certain variations, the cutter may be semitubular or tubular, and may be configured to cut one portion of a tether when the tether extends through the lumen, without simultaneously cutting another portion of the tether. By cutting just one portion of the tether, the devices may be relatively unlikely to leave behind small, loose pieces of the tether that may travel to non-target areas of the body. In certain variations, the elongated member and the cutter may each comprise a wall portion having an opening, and when a tether is extended through the opening in each wall portion, movement of the cutter may cut the tether. In this way, a tether may be cut relatively easily. In some variations, the devices may comprise a guard or guide (e.g., comprising a spherical body) that is configured to prevent the cutter from cutting a wall portion of the elongated member. The guard or guide may at least partially surround the cutter and/or may be coupled to the cutter. The guard or guide may be located at a position distal of the cutter, or at another appropriate position (e.g., proximal to the cutter). The presence of a guard or guide in a device may, for example, limit the likelihood of the device becoming damaged by the cutter (e.g., during use). In some variations, a guard or guide may also be used to direct a tether to the cutter. Certain devices described herein may comprise a pushing member comprising a cable having a proximal portion and a distal portion. A guard or guide may be disposed at the distal portion of the cable. The guard or guide may, for example, be configured to contact a wall portion of a device when the wall portion is curved.

Certain devices described here comprise an elongated member comprising a wall portion and a lumen defined by the wall portion, and a cutter disposed within the lumen. In some variations, the wall portion of the elongated member may have an opening therethrough, and the cutter may also have a wall portion having an opening therethrough. When a tether is extended through the openings, movement of the cutter may cut the tether. In certain variations, the wall portion may comprise first and second openings positioned such that a tether, when extended therethrough, crosses the lumen. The tether may, for example, form a substantially diagonal path across the lumen of the elongated member. In some variations, the cutter may be configured to cut a first portion of a tether extending through the first and second openings, without simultaneously cutting a second portion of the tether. This may, for example, limit or prevent the formation of loose pieces of tether within the body when the devices are used to cut one or more tethers in the body. The elongated member may, for example, be a catheter or a surgical tool.

Certain methods of cutting a tether described herein comprise cutting a first portion of the tether using a semitubular or tubular cutter disposed within an elongated member, without cutting a second portion of the tether with the cutter. At least a portion of the tether may be attached to body tissue, such as heart tissue (e.g., mitral valve tissue). In some variations, the tether may be under tension prior to being cut. In certain variations, the tether may be secured (e.g., using a locking element) prior to being cut. The cutter may, for example, be pushed and/or rotated during use.

Some variations of methods described here comprise cutting a tether using a cutter disposed within a lumen of an elongated member. The tether may be cut by moving the cutter relative to the elongated member when the tether is extended through first and second openings in a first wall portion of the elongated member and a second wall portion of the cutter, respectively. Moving the cutter may comprise pulling the catheter proximally and/or advancing the cutter distally (e.g., by pushing the cutter).

Cutters included in the devices described here may have any appropriate configuration. For example, semitubular or tubular cutters may be used. A cutter may comprise a portion comprising a sharpened edge, such as a beveled edge. In certain variations, the devices may comprise a pushing member that is configured to move the cutter (e.g., within a lumen of an elongated member).

Some variations of the devices described here may be used to lock a tether (e.g., prior to cutting the tether). In certain variations, the devices may comprise a locking element configured to secure a tether, and an elongated member, such as a catheter, that is releasably coupled to the locking element. The locking element may comprise a locking tube, and securing the tether within the locking element may comprise pushing a plug into the locking tube to secure a portion of the tether within the locking tube. In some variations, the locking tube may comprise a shoulder, such as a shoulder with which the elongated member is capable of coupling. The locking element and the elongated member may be coupled to each other when, for example, the device is being used to secure a tether. After the tether has been secured (or, in some cases, as the tether is being secured), the locking element may be decoupled from the elongated member. This decoupling may allow the elongated member to be withdrawn from the tether- securing site while the locking element remains at the site. Different variations of the devices described here may employ different methods and/or components for coupling and decoupling the locking element and the elongated member.

As an example, certain variations of the devices may comprise an elongated member comprising an interlocking feature that couples the elongated member to the locking element when locked, and decouples the elongated member from the locking element when unlocked. In some variations, a coupling line (e.g., a wire) may be used to relatively easily lock and unlock the interlocking feature. Certain variations of devices may not include an interlocking feature, but may include a coupling line (e.g., a wire) that directly couples the elongated member to the locking element (e.g., by being passed through openings in both the locking element and the elongated member). As another example, in some variations, a device may comprise an elongated member that is coupled to a locking element by being fused to the locking element. The fused region may later be broken (e.g., using a pushing member) when it is desired to decouple the elongated member from the locking element. As an additional example, in certain variations of the devices described herein, the locking element and the elongated member may be releasably coupled to each other by at least one electrolytic joint.

Some variations of the devices described here may comprise an elongated member and a locking element releasably coupled to the elongated member (e.g., a distal portion of the elongated member) by a sheath surrounding at least a portion of the elongated member. In certain variations, the sheath may cover the locking element and/or may extend distally of the locking element. The sheath may be configured such that at least partial removal of the sheath from the elongated member decouples the locking element from the elongated member. As an example, the sheath may be configured such that the sheath can be at least partially retracted (e.g., proximally) to decouple the locking element from the elongated member. In some variations, the sheath may have one or more slits and/or openings thereon. For example, in certain variations, the sheath may have a wall portion comprising at least one slit or opening of a size configured for allowing at least a portion of the distal portion of the elongated member to enter the slit or opening (e.g., to pass through the slit or opening). In some variations of the methods described here, the elongated member may become decoupled from the locking element when at least a portion of the distal portion of the elongated member enters the slit or opening (e.g., because the sheath no longer holds the elongated member to the locking element). In certain variations, the slit or opening may be configured so that when the sheath is at least partially retracted (e.g., proximally), the slit or opening opens or becomes wider, and the elongated member releases the locking element. Such decoupling may occur, for example, after the locking element has been used to lock a tether. The sheath may provide the operator with additional control over the timing and process for decoupling the elongated member from the locking element (e.g., preventing premature decoupling, such as decoupling that occurs prior to the locking element locking a tether).

Certain variations of the devices described here may comprise an elongated member, a sheath surrounding at least a portion of the elongated member, and a locking element. The locking element may comprise a locking body having a hollow region, a plug configured to at least partially fit within the hollow region, and at least one locking component configured to at least partially fit over the locking body. The locking body may be releasably coupled to the elongated member. The locking element may be configured to secure a first portion of a tether between the plug and the locking body, and a second portion of the tether between the locking body and the locking component or components. The devices may further comprise a pushing member configured to advance at least a portion of the plug into the hollow region of the locking body, and/or a cutter configured to cut a tether.

In some variations, the locking component or components may comprise at least one protrusion. In some such variations, the locking body may comprise an outer surface having at least one opening and/or groove configured to mate with the protrusion or protrusions. In certain variations, the locking component or components may comprise a plurality of shoulders circumferentially spaced along a perimeter of the locking component or components. The locking body may comprise a wall portion having first and second openings configured for passage of a tether therethrough. In some variations, the locking component or components may be configured to cover the first and second openings when partially fit over the locking body. The locking element may comprise a first locking component configured to cover the first opening in the wall portion and a second locking component configured to cover the second opening in the wall portion. The first opening may be located about 0.5 millimeter to about 5 millimeters from a distal end of the locking body, and/or the second opening may be located about 1 millimeter to about 10 millimeters from a distal end of the locking body.

In some variations, the sheath may comprise a lumen, and the locking component or components may be at least partially disposed (e.g., entirely disposed) within the lumen. Retraction of the sheath may cause the locking component or components to at least partially fit over the locking body. The device may further comprise a pushing member configured to push the locking body such that the locking body at least partially fits within the locking component or components. The locking element may comprise at least one locking component having a circular cross-section. For example, the locking component or components may be in the form of a ring. In some variations, the locking element may comprise at least one locking component having a non-circular cross-section (e.g., a polygonal cross-section).

Some method variations may comprise advancing a tether into a locking body of a locking element further comprising a plug and a locking component. The locking body may be releasably coupled to an elongated member. The methods may also comprise securing a first portion of the tether between the locking body and the plug, and securing a second portion of the tether between the locking body and the locking component. The first portion of the tether may be secured between the locking body and the plug by advancing at least a portion of the plug into a hollow region of the locking body (e.g., using a pushing member). The second portion of the tether may be secured between the locking body and the locking component by advancing at least a portion of the locking body into the locking component. Some variations of the methods may further comprise cutting the tether.

Some of the devices described here may be configured to both lock and cut a tether. For example, certain devices may comprise both a cutter and a locking element that is configured to secure a tether. Thus, a tether may be both locked and cut using a single device. As an example, some variations of devices comprising an elongated member and a cutter may also comprise a locking element that is releasably coupled to a distal portion of the elongated member. The locking element may comprise a locking tube for receiving a plug, and a plug that is configured to fit within the locking tube (e.g., forming an interference fit within the locking tube). The plug may, for example, compress a portion of a tether within the locking tube when the plug enters the locking tube. This may help to lock the tether within the locking tube.

The methods and devices described here may be used to lock and/or cut one tether, or multiple tethers. When multiple tethers are locked and/or cut, the tethers may be locked and/or cut simultaneously, or in succession. In some variations, some tethers may be cut simultaneously, while in other variations, the tethers may be cut in succession.

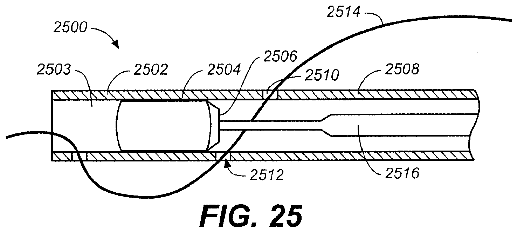

Embodiments of apparatus in accordance with the present invention will now be described, by way of example only, with reference to the accompanying drawings. In the drawings Figs. 25, 31A-31D and 38A-38D depict the subject-matter claimed in claim 1. The remaining drawings nonetheless disclose subject-matter that represents technical background that is useful for understanding the device of the present invention.

### BRIEF DESCRIPTION OF THE DRAWINGS

FIGS. 1A and 1B illustrate the tightening or compressing of tissue of a subject using a tether.
FIG. 2 shows a variation of a device that may be used to lock a tether.
FIGS. 3A-3C show another variation of a device that may be used to lock a tether, and illustrate a method for decoupling certain components of the device from each other.
FIG. 3D shows an additional variation of a device that may be used to lock a tether.
FIGS. 4A-4D show a variation of a device that may be used to lock a tether, and illustrate a method for decoupling certain components of the device from each other.
FIGS. 5A-5D show a variation of a device for locking a tether.
FIG. 6A is a perspective view of a variation of a device that may be used to lock a tether, and FIG. 6B is an enlarged view of region 6B of FIG. 6A.
FIG. 7A is a perspective view of a variation of a device that may be used to lock a tether, FIG. 7B is a side view of the device of FIG. 7A, FIG. 7C is a side schematic view of the device of FIGS. 7A and 7B, and FIG. 7D is a cross- sectional view of the device of FIGS. 7A-7C, taken along line 7D-7D in FIG. 7B.
FIGS. 7E-7H show different variations of components of devices that may be used to lock a tether.
FIG. 8 is a side cross-sectional view of a variation of a device that may be used to lock a tether.
FIGS. 9A-9D illustrate a variation of a method for decoupling a locking element of a locking device from a coupling tube of the locking device.
FIGS. 10A and 10B are side cross- sectional views of a variation of a device that may be used to lock a tether.
FIG. 11A-11F illustrate variations of a device and a method that may be used to lock a tether.
FIGS. 12A and 12B are cross- sectional views of variations of components of tether-locking devices, and FIGS. 12C and 12D show variations of mandrels for use in forming components of tether-locking devices.
FIGS. 13A and 13B depict additional variations of components of tether-locking devices.
FIGS. 14A-14D are cross-sectional views of variations of components of tether-locking devices.
FIG. 15A is a side view of a variation of a component of a tether- locking device, and FIG. 15B is a side cross-sectional view of the component of FIG. 15A.
FIGS. 15C and 15D are side views of a variation of a tether-locking device including the component of FIGS. 15A and 15B.
FIGS. 16A and 16B are top and side partial cross-sectional views, respectively, of a variation of a device that may be used to lock a tether.
FIGS. 17A and 17B are top and side partial cross-sectional views, respectively, of another variation of a device that may be used to lock a tether.
FIGS. 18A and 18B show a variation of a tether-locking device.
FIGS. 19A and 19B are side cross-sectional views of a variation of a device that may be used to lock a tether.
FIGS. 20A and 20B are illustrative variations of devices for loading tethers into catheters.
FIG. 21A shows a device for locking a tether and a device for loading a tether into the tether-locking device.
FIG. 21B shows a tether-locking device with a detachable locking element, and FIG. 21C shows the locking element of FIG. 21B, after the locking element has been detached from the rest of the tether-locking device.
FIG. 22A is a perspective view of a tether-locking catheter, and FIG. 22B is a cross-sectional view of the tether-locking catheter of FIG. 22A, taken along line 22B-22B.
FIGS. 23A-23E are side views of different routing configurations of tethers in tether-locking catheters.
FIGS. 24A-24F illustrate various examples of devices that may be used to cut a tether.
FIG. 25 is a side view in partial cross-section of a variation of a device for cutting a tether.
FIGS. 26A-26C are side views of different routing configurations of tethers in tether-cutting catheters.
FIGS. 27A and 27B depict additional examples of devices that may be used to cut a tether.
FIG. 28 is a side cross-sectional view of a variation of a device that may be used to cut a tether.
FIG. 29 is a side cross-sectional view of another variation of a device that may be used to cut a tether.
FIG. 30A is a side view of a variation of a device that may be used to cut a tether, FIG. 30B is a front view of the device of FIG. 30A, taken from line 30B-30B, FIG. 30C is a side cross- sectional view of the device as shown in FIG. 30B, taken along line 30C-30C, and FIG. 30D is a cross-sectional view of the device in FIG. 30A, taken along line 30D-30D.
FIG. 31A is a perspective view of a variation of a tether-cutting catheter, FIGS. 31B and 31C are side and front views of the tether-cutting catheter of FIG. 31A, respectively, and FIG. 31D is a side cross-sectional view of the tether-cutting catheter of FIG. 31C, taken along line 31D-31D.
FIGS. 32A-32D illustrate variations of tubular devices that may be used to cut a tether.
FIGS. 33A and 33B show additional variations of devices that may be used to cut a tether.
FIG. 34 illustrates a variation of a device that may be used to cut a tether.
FIGS. 35A-35D show variations of devices that may be used to cut a tether.
FIG. 36 is a perspective view of an additional variation of a device that may be used to cut a tether.
FIGS. 37A and 37B show different variations of devices that may be used to lock and cut a tether.
FIG. 38A is a perspective view of a variation of a catheter that may be used to lock and cut a tether, FIG. 38B is a side view of the catheter of FIG. 38A, and FIG. 38C is a side cross-sectional view of the catheter of FIG. 38B, taken along line 38C-38C.

### DETAILED DESCRIPTION

Described here are methods and devices for locking and/or cutting at least one tether (e.g., after the tether has been tensioned to tighten or compress tissue). The devices and methods described here may be used in any appropriate procedures and locations for which such tether locking and/or cutting is desired. While not so limited, the devices and methods described here may be used, for example, in Natural Orifice Transluminal Endoscopic Surgery ("NOTES") procedures, heart valve repair procedures (e.g., mitral valve annulus repair procedures), and/or endoscopic procedures (e.g., laparoscopy or arthroscopy). Some of the devices described here may be used to lock or cut a tether, while other of the devices described here may be used to both lock and cut a tether. Specific examples of methods and devices will now be described in further detail below.

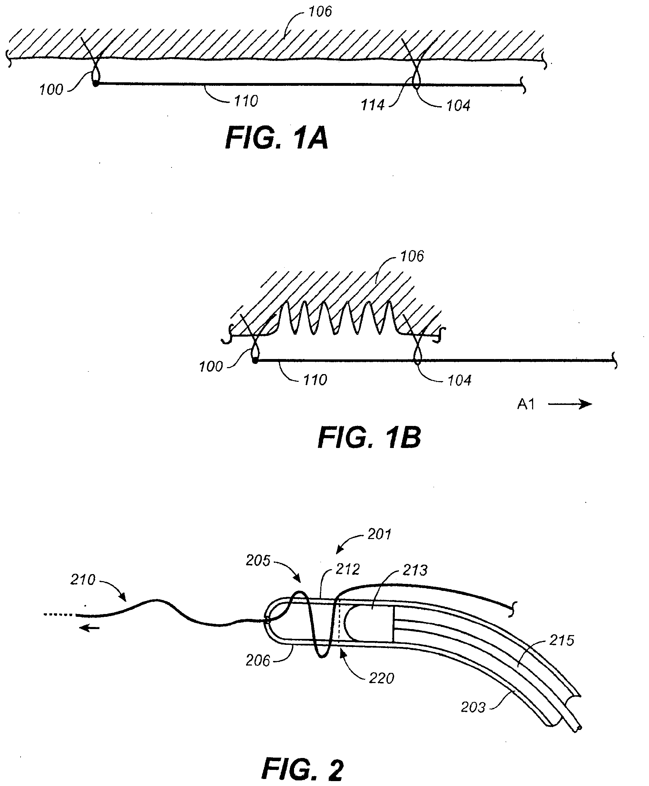

Turning to the figures, FIG. 1A shows two anchors (100) and (104) anchored into tissue (106) of a subject. A tether (110) is fixedly attached to anchor (100), and is threaded through a loop region (114) of anchor (104). As shown in FIG. 1B, when tether (110) is pulled upon in the direction of arrow (A1), a cinching effect results, such that anchors (100) and (104) are brought closer together, and the tissue length between anchors (100) and (104) is reduced. In this way, tissue (106) is tightened or compressed. While two anchors are shown in FIGS. 1A and 1B, in some cases multiple anchors may be used. Additionally, the anchors may all have the same size and shape, or may have different sizes and/or shapes. After tether (110) has been tensioned by a desired amount, tether (110) may be locked to maintain the tension, and in some cases, excess portions of tether (110) may be cut and removed. Tethers may be tensioned, for example, using one or more tensioning devices.

The above-described process may be used in a wide variety of tissues. For example, in some variations, anchors that are connected to each other by a tether may be deployed into tissue in the region of a mitral valve annulus. The tether may then be pulled upon to provide a cinching effect, which restructures the mitral valve annulus (e.g., to reduce mitral valve regurgitation). Thereafter, a locking device may be used to lock the tether in place, thereby maintaining the cinching effect. Finally, a cutting device may be used to remove excess portions of the tether. Mitral valve repair is described, for example, in U.S. Patent Application Publication Nos. US 2006/0190030 A1, US 2006/0122633 A1, US 2008/0172035 A1, and US 2008/0177380 A1. In certain variations, the above-described process may be used in a heart reshaping procedure, such as a ventricular remodeling procedure that is used to repair a heart experiencing valve dysfunction.

As discussed above, the devices and methods described herein may be used, as appropriate, in any of a number of different sites within the body and/or to assist with any of a number of different types of procedures. As an example, the devices and methods described herein may be used in NOTES procedures. As another example, the devices and methods described herein may be used in heart procedures other than those involving mitral valve repair. For example, they may be used to repair an aortic valve or a tricuspid valve, or to secure a prosthetic heart valve, or they may be used in heart ports. As another example, the devices and methods may be employed in a procedure in which one or more tethers are used to reinforce an annuloplasty ring. Additionally, the devices and methods described herein may be used, for example, in a variety of open surgical procedures.

Anchors for use with the methods and devices described here may be any suitable anchor. The anchors may be made of any suitable material, may be any suitable size, and may be of any suitable shape. The anchors may be made of one material or more than one material. Examples of anchor materials include super-elastic or shape memory materials, such as nickel-titanium alloys and spring stainless steel. Examples of anchor shapes include T-tags, rivets, staples, hooks (e.g., C-shaped or semicircular hooks, curved hooks of other shapes, straight hooks, barbed hooks), multiple looped anchors, clips, and the like. The anchors may be configured to self-expand and self-secure into tissue, but need not be configured in such a fashion. Multiple anchors of the same shape may be used, or multiple anchors having different shapes may be used. Similarly, multiple anchors of the same size may be used, or multiple anchors having different sizes may be used. Illustrative examples of suitable anchors are described in more detail, for example, in U.S. Patent Application Publication Nos. US 2005/0273138 A1, US 2008/0058868 A1, US 2008/0045982 A1, US 2008/0045983 A1, US 2008/0051810 A1, and US 2008/0051832 A1. Moreover, while anchors have been described, any other type of suitable fasteners or implants (e.g., leads, electrodes, etc.) may be used with one or more of the devices and/or methods described here. Additionally, some procedures employing the devices and methods described herein may not involve any anchors or other types of fasteners. As an example, certain variations of the devices and methods described here may be used to lock and/or cut a suture that has been sewn through tissue.

Tethers may be one long piece of material or two or more pieces, and may comprise any suitable material, such as suture, suture-like material, a DACRON® polyester strip, high-density polyethylene (HDPE), or the like. In some variations, tethers may be in the form of monofilament or multifilament textile yarns or fibers. Tethers may also have various braided textile constructions. While a tissue-tightening procedure using one tether has been described, other procedures for modifying tissue may involve the use of multiple tethers, such as 2, 3, 4, 5, or 10 tethers. When multiple tethers are used, at least some of the tethers may be associated with (e.g., fixedly attached to) different anchors, and/or at least some of the tethers may be associated with (e.g., fixedly attached to) the same anchor. The devices and methods described herein may apply to single tether procedures, or to multiple tether procedures. As an example, a locking and/or cutting device may be used to lock and/or cut more than one tether, either simultaneously, or at different times.

As described above, after one or more anchors have been secured and the tether has been tensioned, the tether may then be locked or secured into place to maintain the tension (and, therefore, the cinching effect). Different variations of locking devices are described in further detail below.

For example, FIG. 2 shows a locking device (201) including a locking element (205) comprising a plug (213) and a hollow locking member (206). Hollow locking member (206) is releasably coupled to a tubular elongated member (203) in a distal region of the device. Elongated member (203) may be flexible over all or a portion of its length. As shown in FIG. 2, hollow locking member (206) is in the form of a distal extension of elongated member (203) (i.e., hollow locking member (206) extends beyond the distal end of elongated member (203)). However, in some variations, a locking device may comprise an elongated member and a locking member that is coupled to the elongated member, but that does not form a distal extension of the elongated member. Referring again to FIG. 2, hollow locking member (206) maintains the profile of elongated member (203), and may share a common wall with the elongated member. In some cases, though, a locking device may comprise an elongated member and a locking member that is smaller or larger than the elongated member in profile. Alternatively or additionally, the elongated member and the locking member may not share a common wall.

While the device shown in FIG. 2 is configured as a catheter, other configurations may be used. Moreover, the device may be scaled up (e.g., for use in a surgical procedure) or down (e.g., for use in a minimally invasive procedure), depending, for example, on the requirements of the particular procedure in which the device is to be used.

As shown in FIG. 2, a tether (210) is threaded through the distal region of locking device (201), particularly through hollow locking member (206). Although any suitable locking element may be included as part of a locking device, locking element (205) locks a tether when plug (213) is advanced into hollow locking member (206) such that the tether is secured between the plug and a wall of the locking member (e.g., as a result of an interference fit between the plug and the locking member). As shown, tether (210) is threaded through multiple openings in the wall (212) of hollow locking member (206). However, in some variations, a tether may be threaded through only one opening in a wall of a locking member. Alternatively or additionally, a tether may pass through one or more openings (e.g., passages or holes) in one or more other locations of a locking device (e.g., distally of the locking element). In certain variations, one or more openings through which a tether is routed may be radiused (e.g., to enhance passage of the tether through the opening or openings).

Until the locking element is secured, the device may be moved along the tether (e.g., by sliding), or the tether may be pulled through the device. Thus, the tether may be used to provide a cinching effect by sliding the device distally down the tether. The openings through the device shown in FIG. 2 may be positioned such that the device can still easily slide along the tether. In some variations, the tether may be threaded into the locking element in such a way that it winds in and out of the locking element, as suggested by FIG. 2.

The tether may be threaded or coupled to the device, for example, by the user. For example, and as described further below, a lasso may be threaded through the openings in the device. The lasso may then be used to engage the tether and to thread the tether through the openings (e.g., by pulling on the opposite end of the lasso).

In some variations, the device may be slid along the tether until the tether has been pulled by the desired amount through the anchors, at which point the tether may be secured into position using the locking element. For example, and as described above, tether (210) of FIG. 2 may be secured into position by pushing plug (213) into hollow locking member (206) of locking element (205) (e.g., as a result of an interference fit between the plug and the locking member). In the variation shown in FIG. 2, plug (213) secures tether (210) by compressing at least a portion of the tether between the plug and the inner walls of hollow locking member (206).

The plug and/or hollow locking member of the locking element may comprise one or more features that limit the likelihood of the plug being released from the hollow locking member. For example, the plug and/or hollow locking member may include adhesive, glue, or cement, and/or may be at least partially deformable so that once the plug has been inserted into the hollow locking member, the plug is retained within the locking member. As an example, the plug may comprise a material which is compressible or elastic to aid in locking the plug into the locking member. In certain variations, the plug may have polygonal (e.g., hexagonal) sides that interact with the inner surface of the locking member. The plug may be solid or hollow. The plug may have bumps, dimples, ribs, grooves, or holes on its surface to increase friction with the tether. The locking member may also include or comprise structures (e.g., rims, brackets, etc.) to help hold the plug in the locked configuration. In some variations, the locking member itself may alternatively or additionally be polygonal in cross-section. In certain variations, the plug and the locking member may have corresponding geometries, as described below. In some variations of devices, the plug and the locking member may each include different features that enhance the retention of the plug in the locking member.

The device shown in FIG. 2 further includes a pushing member (215) for pushing plug (213) into position to secure tether (210) within hollow locking member (206). The pushing member (shown in FIG. 2 as a rod, although other suitable forms of pushing members may be used) may be slidable within the lumen of the device. In some variations, the pushing member may include guides (e.g., that guide the pushing member's direction) and/or stops (e.g., that limit the distance traveled by the pushing member and/or the force applied by the pushing member). Thus, there may be motion-limiting features on the device and/or pushing member to prevent the pushing member from being pushed too far forward, or from applying too much force, which could disturb either the locking element or the tissue (e.g., after separation of the locking element from the rest of the device).

As described above, a locking element may be releasably coupled to the rest of a device. Any appropriate method may be used to provide such a releasable coupling. In some variations, the locking element (or a portion thereof) may include a releasable coupling region, such as a region that can be separated or broken to release the locking element from the rest of the device. As an example, a locking element may be frangibly connected to the rest of a device, and may be decoupled from the device by breaking the frangible connection. For example, a locking element may be fused to another portion of the device (e.g., a distal portion of an elongated member). The fused region may later be broken to decouple the locking element from the other portion of the device. The amount of heat and/or pressure that is applied during the fusion process, as well as the number of fused regions and their locations, may be selected so that a specific amount of force can be applied to the fused regions to break them.

Different regions of a locking device may comprise different materials, or may comprise the same material. In some variations, a locking device may comprise a locking element formed of a first material, another portion formed of a second material, and a fused region between the locking element and the other portion that is formed of a third material (or combination of materials). Using different materials for different regions of a locking device may be advantageous if the different regions have different material requirements. For example, a more distal region of the device may be formed of one or more materials that provide relative flexibility, while a more proximal region may be formed of one or more materials that provide relative stiffness, or vice-versa. Moreover, while locking devices comprising one or more fused regions and multiple different materials have been described, some variations of locking devices may comprise fused regions and may be formed entirely of one material or combination of materials, and other variations of locking devices may comprise multiple different materials (e.g., 2, 3, 4, or 5 different materials) without comprising any fused regions.

In certain variations, a locking device comprises a detachable locking element that is coupled to the rest of the device by a structurally weakened region that is, for example, scored, etched, perforated, fractured, creased, slotted, and/or dimpled. An example of a perforated region (220) is shown in FIG. 2. The locking element may be made of the same material as the rest of the device, or the locking element and the rest of the device may be made of different materials. When a sufficient amount of force is applied to the structurally weakened region, the locking element may become separated from the rest of the device. Force may be applied to the structurally weakened region using, for example, a pushing member or any suitable mechanism.

In some variations, a locking element may be releasably coupled to another portion of a locking device via at least one adhesive and/or a friction fit, so that the application of a certain amount of force may cause the locking element to decouple from the other portion of the locking device. Additional non-limiting methods of releasably coupling a locking element to another portion of a locking device include fusing, brazing, soldering, and snap-locking. In some variations of locking devices, two or more different releasable coupling methods may be used in conjunction with each other.

As described above, in some variations, a locking element may be controllably decoupled from the rest of a device by applying a force. Force may be applied in any appropriate manner (e.g., pushing on a pushing member, hydraulic force (using saline, water, or the like), magnetic force, pressurized gas, etc.). For example, the same pushing member (215) of FIG. 2, used to push plug (213) and secure the locking element, may also be used to decouple the locking element from the rest of the device (e.g., by pushing the pushing member with additional force). In some variations, one force applicator (e.g., a pushing member) may be used to secure the locking element and another force applicator (e.g., a second pushing member) may be used to decouple the locking element from the rest of the device.

The amount of force required to decouple a locking element from the rest of a device may be predetermined. In variations where the same force applicator (e.g., a pushing member, fluid line, magnet, etc.) is used both to lock the tether and to decouple the locking element, the force required to decouple the locking element may be greater than the force required to secure the locking element and thereby lock the tether. For example, a device may be configured for its locking element to decouple after the application of greater than about 8.9N (2 lbs) of force, greater than about 13.3N (3 lbs) of force, greater than about 17.8N (4 lbs) of force, greater than about 22.2N (5 lbs) of force, greater than about 44.5N (10 lbs) of force, greater than about 89N (20 lbs) of force, or between about 8.9N (2 lbs) and about 22.2N (5 lbs) of force. The amount of force that is needed to decouple a locking element from the rest of a locking device can depend on any of a number of different factors. Such factors may include, for example, the thickness of the coupling region, the material or materials that form the coupling region, and/or the location of scoring, perforations, or other weakened points in the coupling region. In some cases, the amount of force that is required to decouple a locking element from the rest of a locking device, as well as the way in which the force is applied to decouple the locking element, may be controlled to prevent damage to the locking element, the tether, the anchors, and/or the surrounding tissue.

While the application of force to decouple a locking element from the rest of a locking device has been described, other decoupling methods may alternatively or additionally be employed. As an example, a locking element may be decoupled by cutting a joint between the locking element and the rest of the device using, for example, a cutter. In some variations, the cutter may be in the form of a shearing blade that slides to shear the joint between the locking element and the rest of the device. In certain variations, a cutter that cuts the connection between a locking element and the rest of a locking device may also be used to cut a tether being secured by the locking device. For example, the cutter may cut both the tether and the joint in a combined manner, thus completely releasing the locking element with the tether severed.

Other methods and/or devices may be used to couple and decouple a locking element with another portion of a locking device.

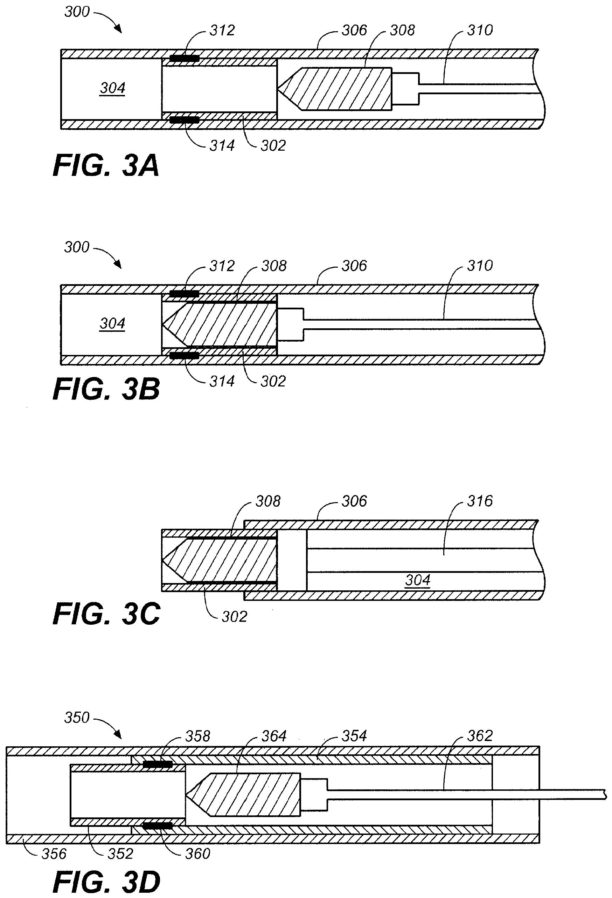

As an example, FIGS. 3A-3C illustrate the decoupling of a locking element from a tubular elongated member (e.g., a catheter) by breaking one or more regions where the locking element and the elongated member are fused to each other. As shown in FIG. 3A, a locking device (300) comprises a locking tube (302) disposed within a lumen (304) of a tubular elongated member (306). A plug (308) is also disposed within lumen (304), proximal to the locking tube. The locking tube and the plug together form a locking element. A pushing member (310) is disposed within lumen (304), as well, although some variations of devices may not include a pushing member.

As shown in FIGS. 3A and 3B, locking tube (302) is coupled to elongated member (306) via two fused regions (312) and (314). While two fused regions are shown, any number of fused regions (e.g., 1, 3, 4, 5, 10) may be used as desired. Moreover, the fused regions may be longer or shorter than fused regions (312) and (314) and/or may be in any suitable location of a locking device. Additionally, a locking device may include fused regions of the same size and/or shape, or may include at least some fused regions of different sizes and/or shapes.

FIG. 3B shows pushing member (310) pushing plug (308) into locking tube (302) (e.g., to secure a tether (not shown) within the locking tube). Finally, FIG. 3C shows a different pushing member (316) being used to apply enough force to locking tube (302) to break fused regions (312) and (314), thereby decoupling locking tube (302) from elongated member (306). The locking tube can then be pushed out of the elongated member and left at a locking site while the elongated member is withdrawn.

While FIGS. 3A-3C show different pushing members being used to push the plug into the locking tube and to push the locking tube out of the elongated member, in some cases, the same pushing member may be used for both tasks. Furthermore, in certain variations, a locking tube may not be decoupled from an elongated member using a pushing tube. As an example, in some variations, a locking tube may be pulled out of an elongated member to decouple the locking tube from the elongated member. Locking tubes and elongated members that are coupled to each other by one or more fused regions may be decoupled using any other suitable method. As an example, in certain variations, a locking tube may be decoupled from an elongated member by using a blade to shear one or more fused regions between the locking tube and the elongated member. Additionally, while fused regions have been described, in some variations, components of a locking device, such as a locking tube and an elongated member, may alternatively or additionally be coupled to each other by any other suitable methods (e.g., adhesive-bonding, etc.).

Referring again to FIGS. 3A-3C, fused regions such as fused regions (312) and (314) may be formed, for example, by applying heat to specific regions of locking tube (302) and elongated member (306). This application of heat can cause the material or materials of the locking tube and the elongated member in those regions to melt and combine with each other. Upon cooling, the locking tube and the elongated member may be coupled to each other at the regions that were heated. The number of fused regions and/or the areas of the fused regions may be varied to adjust the force necessary to later break the fused regions. For example, an increase in the number of fused regions and/or in the areas of the fused regions may generally result in a higher force being required to break the fused regions, while a decrease in the number of fused regions and/or in the areas of the fused regions may generally result in a lower force being required to break the fused regions. In some variations, a fused region between a locking tube comprising a first material (e.g., a polymer) and an elongated member comprising a second material (e.g., another polymer) may be formed by using a third material (e.g., a third polymer) to secure the two materials together. For example, the third material may be disposed between the first and second materials, and may be heated to fuse to the first material on one side and the second material on the other side. Such a third material may be employed, for example, when the first and second materials do not readily fuse to each other, but do readily fuse to the third material.

Thus, fused regions may be formed relatively easily, and may provide a relatively efficient way to releasably couple a locking tube to one or more other components of a locking device. Moreover, while the use of fused regions to couple a locking tube and an elongated member has been described, in some device variations, fused regions may be used to couple other components of a locking and/or cutting device to each other. For example, in some variations, a locking device may comprise a sheath surrounding an elongated member and a locking tube having a proximal section that is coupled to the elongated member. The sheath may be temporarily coupled to the elongated member and/or to the locking tube using one or more fused regions.

In certain variations, a locking device may comprise a sheath that is not temporarily coupled to other components of the locking device using one or more fused regions. For example, FIG. 3D shows a locking device (350) comprising a locking tube (352) and an elongated member (354) disposed within a sheath (356). Locking tube (352) is temporarily coupled to elongated member (354) by two fused regions (358) and (360). During use, a pushing member (362) may be used to push a plug (364) into locking tube (352). The same pushing member, or a different pushing member, may then be used to break the fused regions between the locking tube and the elongated member. Sheath (356) may then be removed from the locking tube (e.g., by proximally withdrawing the sheath away from the locking tube), thereby leaving the locking tube at the target site. Sheath (356) may serve, for example, to keep all of the other components of the locking device (e.g., locking tube (352), plug (364)) together in one place.

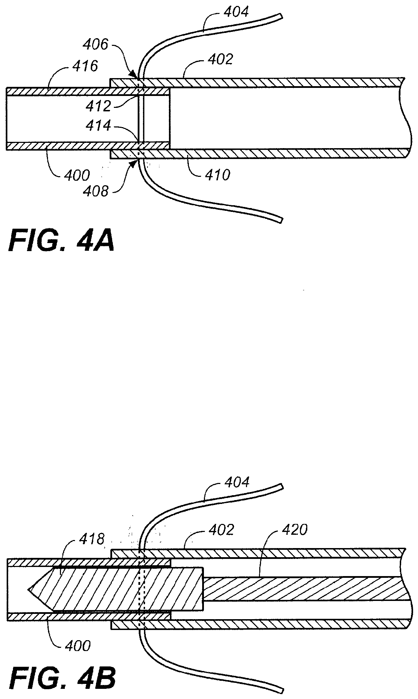

As another example, FIGS. 4A-4D illustrate the decoupling of a locking element from a tubular elongated member via the removal of a coupling line that releasably couples the locking element to the elongated member. As shown in FIG. 4A, a locking tube (400) is coupled to a tubular elongated member (402) by a coupling line (404). Coupling line (404) is threaded through two openings (406) and (408) in a wall portion (410) of the elongated member, as well as two openings (412) and (414) in a wall portion (416) of the locking tube. However, in some variations of devices, a coupling line may be threaded through fewer openings in an elongated member wall portion and/or a locking tube wall portion, or may be threaded through more openings in an elongated member wall portion and/or a locking tube wall portion. Coupling line (404) may be in the form of a cable, thread, wire, suture, tether, etc., and may be made of any appropriate material or materials.

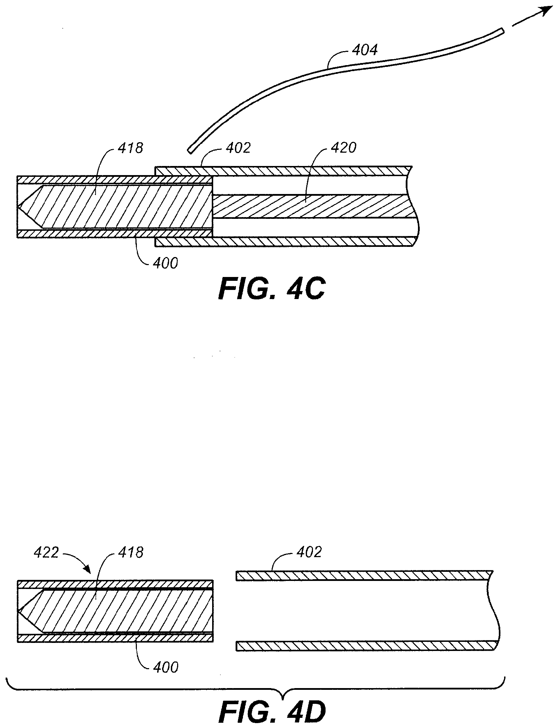

FIG. 4B shows a plug (418) being pushed into locking tube (400) by a pushing member (420) (e.g., to secure a tether (not shown) between the plug and the locking tube). Referring now to FIG. 4C, after the plug has been pushed into the locking tube (or, in some cases, while the plug is being pushed into the locking tube), coupling line (404) is removed from both the locking tube and the elongated member. As shown in FIG. 4D, locking tube (400) and plug (418), which together form a locking element (422), are then decoupled from elongated member (402) using, for example, pushing member (420) or another appropriate pushing member (not shown).

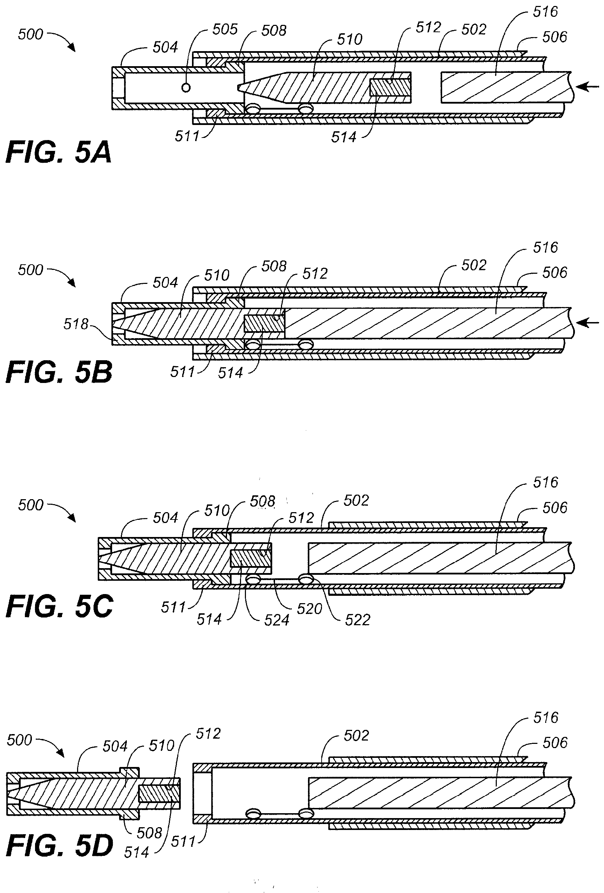

FIGS. 5A-5D illustrate a method of locking a tether using another variation of a locking device. Referring first to FIG. 5A, a locking device (500) includes a coupling tube (502) having a distal portion that is coupled to a locking element (504). As shown, locking element (504) is in the form of a locking tube having an opening (505) configured for passage of a tether therethrough. While a locking tube is shown, other suitable configurations may be used for a locking element. Locking element (504) can be formed of one or more metals, metal alloys, and/or polymers. As an example, in some variations, locking element (504) is formed of a nylon and bismuth trioxide composite, and includes a layer of polyether block amide, such as PEBAX® polyether block amide.

A sheath (506) surrounds coupling tube (502), as well as a portion of locking element (504). However, in some variations, a sheath may cover the entirety of a locking element, and may even extend distally beyond the locking element. Moreover, in certain variations, a sheath may surround only a portion of a coupling tube. Sheath (506) helps to couple coupling tube (502) to locking element (504) by compressing the coupling tube to the locking element. Additionally, locking element (504) includes a shoulder (508), and coupling tube (502) is configured to latch onto shoulder (508) when sheath (506) compresses coupling tube (502) to locking element (504). As shown, coupling tube (502) comprises a shoulder (511) that latches to shoulder (508). While shoulders (508) and (511) are shown as generally angular, in some variations, a locking element shoulder and/or a coupling tube shoulder may be ramp-shaped, or may have any other suitable shape. A ramp- shaped coupling tube shoulder may, for example, provide for relatively easy decoupling of the coupling tube from the locking element when such decoupling is desired.

Locking device (500) is configured such that if sheath (506) is proximally retracted, locking element (504) is decoupled from coupling tube (502). However, in certain variations, a sheath may be proximally retracted, while a coupling tube and locking element are distally pushed upon, in order to decouple the locking element from the coupling tube. Alternatively or additionally, the coupling element and locking tube may be distally pushed upon before and/or after the sheath is proximally retracted. Any other suitable methods for decoupling the locking element from the coupling tube may also be employed.

As shown in FIG. 5A, a plug (510) is disposed within coupling tube (502), and has a generally missile- shaped configuration, although other appropriate configurations (e.g., a plug having any appropriate geometry, such as a plug in the shape of a cylinder or a plug having a hexagonal cross-section) may also be used. The plug can be formed of any appropriate materials, such as one or more polymers, and may in some variations be relatively rigid. In certain variations, plug (510) may be formed of a nylon and bismuth trioxide composite. As shown in FIG. 5A, plug (510) includes a bore (512) containing a radiopaque marker (514). This may allow for ready viewing of the plug via X- ray fluoroscopy. A pushing member (516) is also disposed within coupling tube (502), and may be used to push plug (510) into locking element (504).

During use of locking device (500), a tether (not shown) may be threaded through locking element (504) and coupling tube (502). Any appropriate method may be used to thread the tether including, for example, one or more of the methods described above. As an example, a lasso may be used to capture the distal end of the tether, and to thread the tether first through opening (505), and then through coupling tube (502). In some methods, the locking device may be advanced along the tether to a desired position. As shown in FIG. 5B, once the tether has been threaded through locking element (504) and coupling tube (502), pushing member (516) may be advanced toward the distal end of the locking device. This advancement of pushing member (516) pushes plug (510) into locking element (504), compressing the tether between plug (510) and the inner walls of locking element (504) (e.g., as a result of an interference fit between the plug and the locking element). Because coupling tube (502) engages shoulder (508) of locking element (504), a resistive force is provided during plug advancement. This resistive force may help to limit the likelihood of locking element (504) becoming prematurely decoupled from coupling tube (502), as a result of the advancement of pushing member (516). A step (518) at the distal end of the locking element may prevent the plug from exiting the locking element.

Referring now to FIG. 5C, after plug (510) has been pushed into locking element (504), sheath (506) may be proximally retracted. Prior to being proximally retracted, sheath (506) compresses coupling tube (502) to locking element (504), thereby engaging coupling tube (502) with the shoulder (508) of locking element (504) and coupling the coupling tube to the locking element. However, once sheath (506) has been proximally retracted, this compressing force is no longer present. Coupling tube (502) is configured such that in the absence of this compressing force, coupling tube (502) no longer forms a tight fit around locking element (504). Rather, the removal of the compressing force allows coupling tube (502) to assume a more relaxed configuration, essentially opening up and thereby disengaging coupling tube (502) from shoulder (508) of locking element (504). As a result, coupling tube (502) and locking element (504) are decoupled from each other. This assumption of a more relaxed configuration by coupling tube (502) is enhanced by the presence of a slit (520) in the distal portion of the coupling tube, as well as two openings (522) and (524) along the slit that provide stress relief. While not shown, in some variations, a coupling tube may include more than one slit in its distal portion. Moreover, while openings (522) and (524) are circular, in certain variations, a coupling tube may alternatively or additionally include one or more non-circular (e.g., rectangular, triangular, etc.) openings.

Referring finally to FIG. 5D, and as discussed above, the proximal retraction of sheath (506) causes locking element (504) to be released from coupling tube (502). Plug (510), which was previously pushed into locking element (504), is released along with locking element (504). The locking element and plug, now separated from the other elements of the locking device, remain within the body, securing the tether, while the other elements of the locking device are removed from the body. In this way, sheath (506) may function as a safety mechanism, preventing locking element (504) from being released prematurely, and providing the operator with enhanced control over the release of locking element (504).

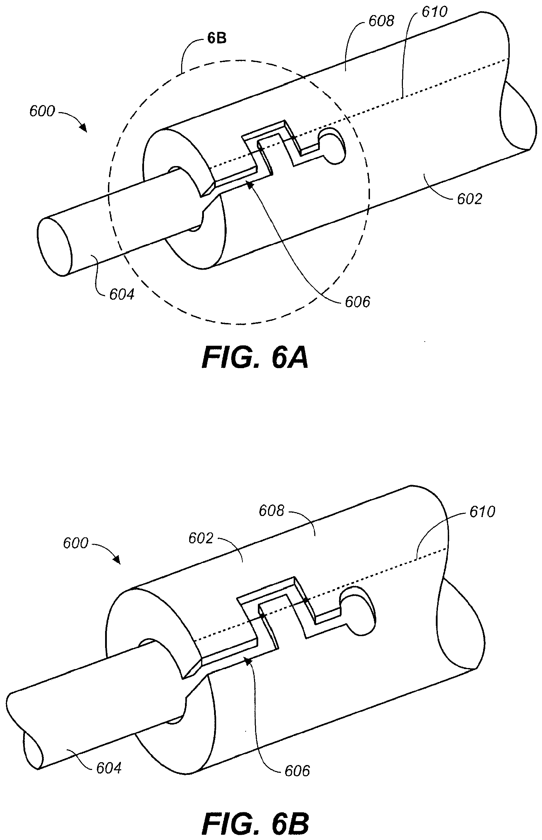

An additional example of a locking device is shown in FIGS. 6A and 6B. As shown there, a locking device (600) includes a tubular elongated member (602) that is coupled to a locking element (604). Elongated member (602) has an interlocking feature (606) cut into its wall (608). Interlocking feature (606) is held locked by a coupling line (610) that is routed through the interlocking feature. When coupling line (610) is pulled out, interlocking feature (606) is released, thereby eliminating the hoop strength of elongated member (602). This causes the elongated member to decouple from locking element (604) (e.g., by disengaging from a shoulder feature (not shown) on the locking element). While one coupling line is shown, in some variations, an interlocking feature may be locked and unlocked using multiple (e.g., 2, 3, 4, or 5) coupling lines.

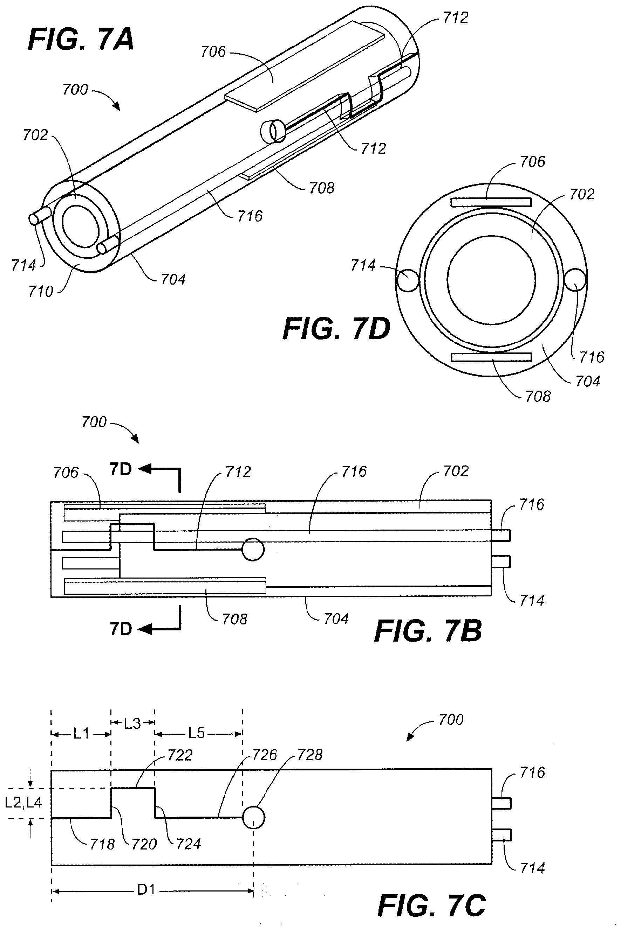

FIGS. 7A-7D also show an interlocking feature in a locking device. As shown in FIGS. 7A-7D, a distal portion (700) of a locking device comprises a locking tube (702) disposed within a tubular elongated member (704). While not shown, tubular elongated member (704) may, for example, extend proximally for an additional length to form the rest of the locking device, or may be coupled to another elongated member to form the rest of the locking device. Other configurations may also be used.

As shown in FIGS. 7A, 7B, and 7D, two pieces of Nitinol flat wire (706) and (708) are embedded within the wall (710) of elongated member (704) to provide the elongated member with enhanced rigidity. While Nitinol has been described, other materials may be used. Moreover, in some variations, the locking device may not include flat wire, or may include only one piece of flat wire or more than two pieces of flat wire.

Elongated member (704) includes two interlocking features in its wall (710). While FIGS. 7A-7D only show one interlocking feature (712), a corresponding interlocking feature is located on the other side of the elongated member. However, some variations of locking devices may include only one interlocking feature, or may include multiple interlocking features having different configurations.

The interlocking features in elongated member (704) are comprised of slits that are cut into wall (710), although different types of interlocking features are possible. For example, an interlocking feature may be formed of a combination of polygonal openings. As shown in FIGS. 7A and 7B, the portions of wall (710) on either side of the slits are held together (and thereby kept in a locked configuration) by two wires (714) and (716) that extend through lumens within wall (710). The use of wires that extend through lumens in the wall of elongated member (704) may allow the elongated member to maintain a relatively low profile. While wires have been described, any other suitable coupling lines (e.g., cables, threads, sutures, tethers, etc.) may be used. Moreover, certain variations of devices may comprise only one coupling line, or multiple (e.g., 2, 3, 4, 5) coupling lines. In device variations comprising multiple coupling lines, the coupling lines may be the same type of coupling line, or may be different from each other. For example, a device may include one coupling line in the form of a wire, and a second coupling line in the form of a suture.

Locking tube (702) is decoupled from elongated member (704) by withdrawing wires (714) and (716) (e.g., using button sliders on the handle of the locking device) and thereby unlocking the interlocking features. In some cases, this unlocking alone may be sufficient to release the locking tube from the elongated member. In other cases, additional assistance (e.g., pushing the locking tube with a pushing member) may be required to release the locking tube from the elongated member.

As shown in FIG. 7C, interlocking feature (712) has a configuration comprising a first slit portion (718) having a length L1, a second slit portion (720) having a length L2, a third slit portion (722) having a length L3, a fourth slit portion (724) having a length L4, a fifth slit portion (726) having a length L5, and an opening (728). In some variations, length L1 may be from about 1 millimeter to about 5 millimeters, length L2 may be from about 1 millimeter to about 4 millimeters, length L3 may be from about 1 millimeter to about 5 millimeters, length L4 may be from about 1 millimeter to about 4 millimeters, and/or length L5 may be from about 1 millimeter to about 5 millimeters. Moreover, the diameter of opening (728) may, for example, be from about 0.25 millimeter to about 4 millimeters. While a circular opening has been shown, any suitable non-circular openings may be used. Interlocking feature (712) also has a dimension D1 which may be from about 4 millimeters to about 10 millimeters. Interlocking feature (712) has a particular shape or configuration; however, interlocking features having other shapes or configurations may be employed.

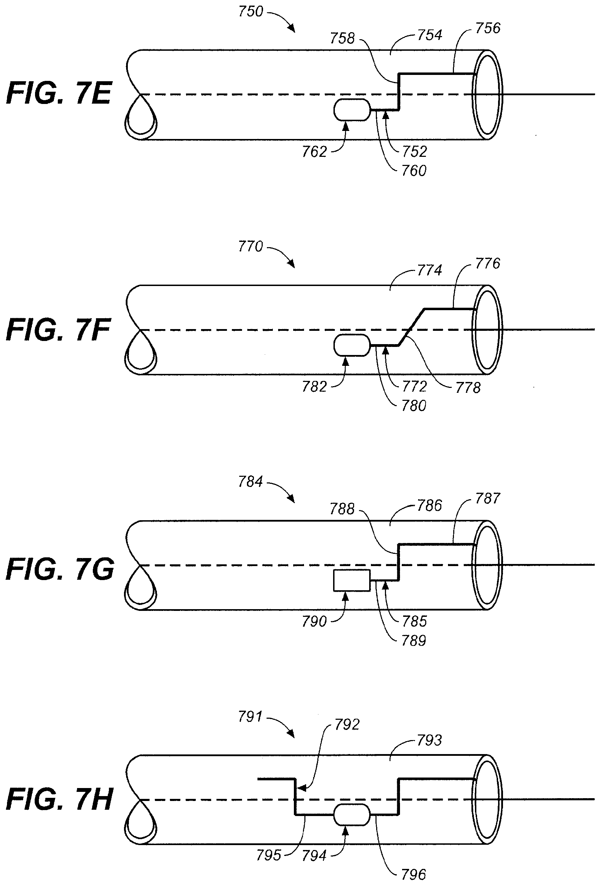

For example, FIGS. 7E-7H show different variations of elongated members, each having a different interlocking feature. As shown in FIG. 7E, an elongated member (750) of a locking device includes an interlocking feature (752) in its wall (754). Interlocking feature (752) comprises a first slit portion (756), a second slit portion (758) orthogonal to the first slit portion, a third slit portion (760) orthogonal to the second slit portion, and an oval opening (762). However, FIG. 7F shows an elongated member (770) of a locking device that includes an interlocking feature (772) of a different configuration in its wall (774). As shown there, interlocking feature (772) comprises a first slit portion (776), a second slit portion (778), a third slit portion (780), and an oval opening (782). None of the slit portions is orthogonal to any of the other slit portions. FIG. 7G shows an elongated member (784) of a locking device including an interlocking feature (785) of yet another configuration in its wall (786). As shown in FIG. 7G, interlocking feature (785) comprises a first slit portion (787), a second slit portion (788) orthogonal to the first slit portion, a third slit portion (789) orthogonal to the second slit portion, and a rectangular opening (790). An interlocking feature configuration may be selected, for example, based on the number and/or type of wires that are used to lock the interlocking feature, as well as the dimensions of the wires. Alternatively or additionally, an interlocking feature configuration may be selected to provide relatively easy unlocking of the interlocking feature (e.g., by retraction of one or more wires).

Some variations of interlocking features may comprise an opening and at least two slits extending from the opening. As an example, FIG. 7H shows an elongated member (791) of a locking device that includes an interlocking feature (792) in its wall (793). The interlocking feature comprises an opening (794) and multiple slit portions, including a first slit portion (795) and a second slit portion (796), both of which extend from the opening. Still other configurations of interlocking features may be used as desired.

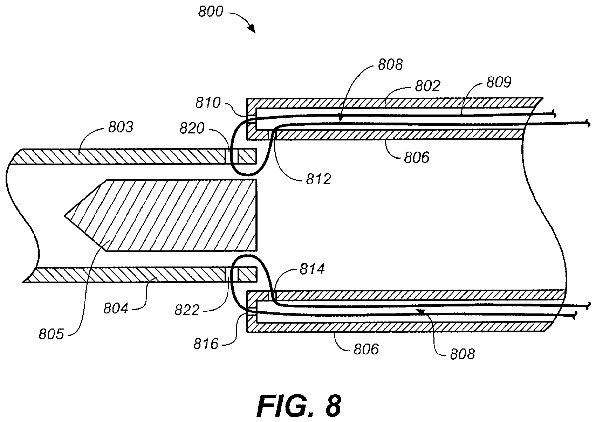

Further variations of locking devices may be used. For example, FIG. 8 shows a locking device (800) including a catheter (802) and a locking element (804) that is coupled to catheter (802). Locking element (804) includes a locking tube (803) and a plug (805). Catheter (802) comprises a wall (806) having an interior region (808). A coupling line (809), such as a cable, thread, wire, suture, tether, etc., is routed through interior region (808) of wall (806), briefly exiting interior region (808) via openings (810), (812), (814), and (816), in wall (806). Coupling line (809) also is looped through openings (820) and (822) in locking tube (803), thereby coupling catheter (802) to locking element (804). In certain variations of devices, the locking tube may comprise a wall having an interior region through which the coupling line is routed, either as an alternative to, or in addition to, the catheter wall having such an interior region. Moreover, different coupling line routing configurations may be used to couple a locking tube to a catheter or other elongated member. Referring back to FIG. 8, decoupling of catheter (802) from locking element (804) may be achieved, for example, by cutting coupling line (809) and/or pulling coupling line (809) out of device (800).

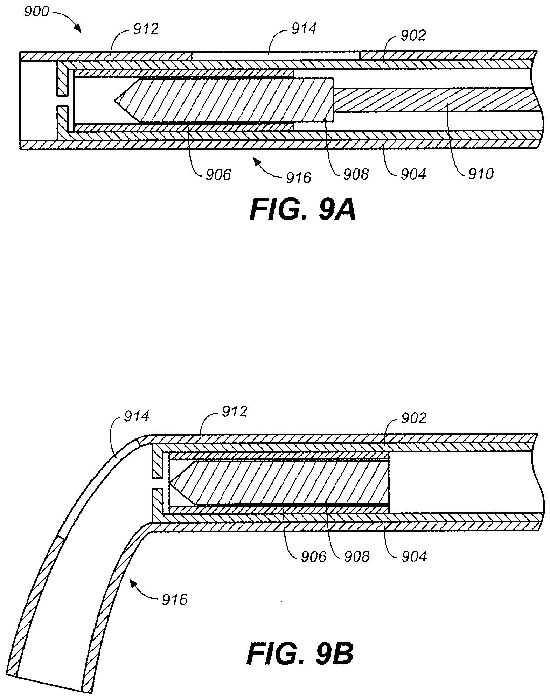

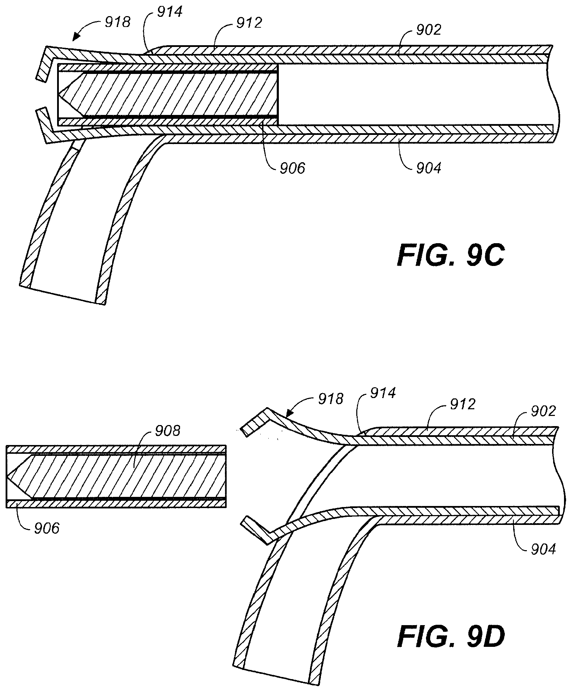

FIGS. 9A-9D show another variation of a locking device. As shown there, a locking device (900) comprises a coupling tube (902) disposed within a sheath (904), and a locking tube (906), plug (908) and pushing member (910) disposed within coupling tube (902). Sheath (904) includes a wall portion (912) having an opening (914) in it. In FIG. 9A, plug (908) is being pushed into locking tube (906) using pushing member (910). As shown in FIG. 9B, after the plug has been pushed into the locking tube, coupling tube (902) is proximally withdrawn past the location of opening (914). The distal portion (916) of sheath (904), which is preformed to curve, had previously been relatively straight because of the support of coupling tube (902), as shown in FIG. 9A. However, as coupling tube (902) is withdrawn, distal portion (916) assumes its natural curved shape. As shown in FIG. 9C, the distal portion (918) of coupling tube (902) is then pushed out of sheath (904) through opening (914) using, for example, a pushing member (not shown). When the distal portion of the coupling tube is no longer constrained by the sheath, it opens up, as shown in FIG. 9D, thereby releasing locking tube (906) and plug (908).

While there is a single opening in wall portion (912) of sheath (904), some variations of devices may comprise sheaths that have multiple openings in their wall portions. Additionally, certain variations of devices may comprise sheaths that have one or more slits in their wall portions, either in addition to, or as an alternative to, having one or more openings. Moreover, while distal portion (916) of sheath (904) is described as having a preformed curved shape, in some device variations, a sheath distal portion may have a relatively straight shape, even when a coupling tube is not disposed within the sheath distal portion.

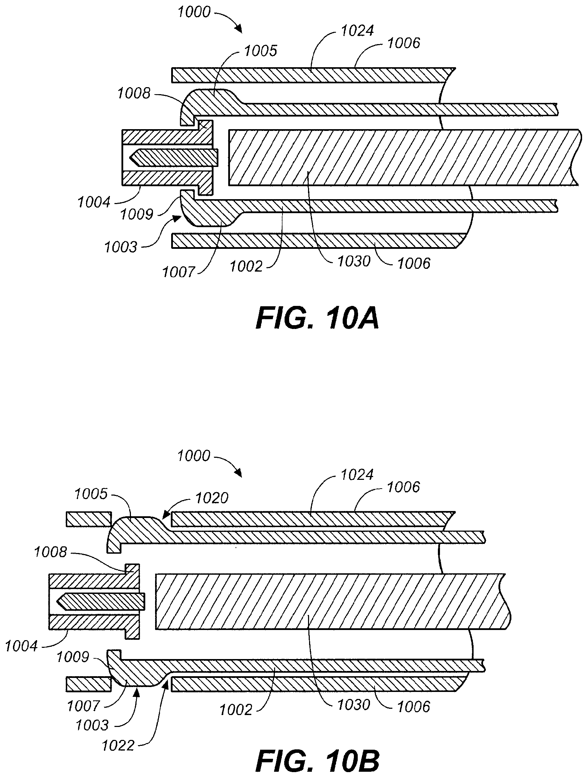

FIGS. 10A and 10B show an additional variation of a locking device. As shown in FIG. 10A, a locking device (1000) includes a coupling tube (1002) having a distal portion (1003) that is coupled to a locking element (1004). Distal portion (1003) includes two rounded protrusions (1005) and (1007). As shown, locking element (1004) is in the form of a locking tube that receives a locking plug, but other suitable configurations may be used.

A sheath (1006) surrounds coupling tube (1002), as well as a portion of locking element (1004). In certain device variations, however, a sheath may completely surround a locking element. Sheath (1006) helps to couple coupling tube (1002) to locking element (1004) by compressing the coupling tube to the locking element. Additionally, locking element (1004) includes a shoulder (1008), and coupling tube (1002) includes a corresponding shoulder (1009) that is configured to latch onto shoulder (1008) when sheath (1006) compresses coupling tube (1002) to locking element (1004).

Locking device (1000) is configured such that if sheath (1006) is rotated (e.g., by about 90°), locking element (1004) is decoupled from coupling tube (1002). This occurs because sheath (1006) includes openings (1020) and (1022) in its wall portion (1024). As the sheath is rotated, openings (1020) and (1022) become aligned with protrusions (1005) and (1007) on coupling tube (1002). Openings (1020) and (1022) provide room for distal portion (1003) of coupling tube (1002) to expand, such that protrusions (1005) and (1007) enter openings (1020) and (1022). This releases the compressive force of coupling tube (1002) on locking element (1004), such that shoulder (1009) of coupling tube (1002) becomes disengaged from shoulder (1008) of locking element (1004). As a result, coupling tube (1002) is decoupled from locking element (1004). While sheath (1006) is described as being rotated, in some variations, coupling tube (1002) may alternatively or additionally be rotated such that protrusions (1005) and (1007) enter openings (1020) and (1022). Moreover, while wall portion (1024) of sheath (1006) is shown as having two openings (1020) and (1022), certain variations of devices may comprise a sheath with a wall portion having only one opening or more than two openings. Similarly, some variations of devices may comprise coupling tubes having only one protrusion or more than two protrusions.

As shown in FIGS. 10A and 10B, locking device (1000) also includes a pushing member (1030). Pushing member (1030) may be used to help coupling tube (1002) expand when openings (1020) and (1022) become aligned with protrusions (1005) and (1007) on coupling tube (1002). More specifically, pushing member (1030) may be pushed distally, thereby pushing against locking element (1004), such that shoulder (1008) of locking element (1004) pushes against shoulder (1009) of coupling tube (1002). This force against shoulder (1009) of coupling tube (1002) may further push coupling tube (1002) toward openings (1020) and (1022). In certain variations, shoulder (1009) of coupling tube (1002) may be ramp-shaped, which may increase this effect.

Other variations of locking devices are also described here. As an example, while sheath (1006) in FIGS. 10A and 10B is rotated to decouple coupling tube (1002) from locking element (1004), in some variations, a sheath may be translated to decouple a coupling tube from a locking element. For example, a sheath may be advanced distally or withdrawn proximally (with or without also rotating the sheath) to align one or more openings in the sheath with one or more protrusions on a coupling tube that is coupled to a locking element. As a result, the protrusions can expand into the openings and thereby allow the coupling tube to become decoupled from the locking element. Moreover, in certain variations, one or more other components of a device may alternatively or additionally be translated and/or rotated to decouple a coupling tube from a locking element. As an example, in some variations, rotation and/or translation of a coupling tube may cause the coupling tube to decouple from a locking element.

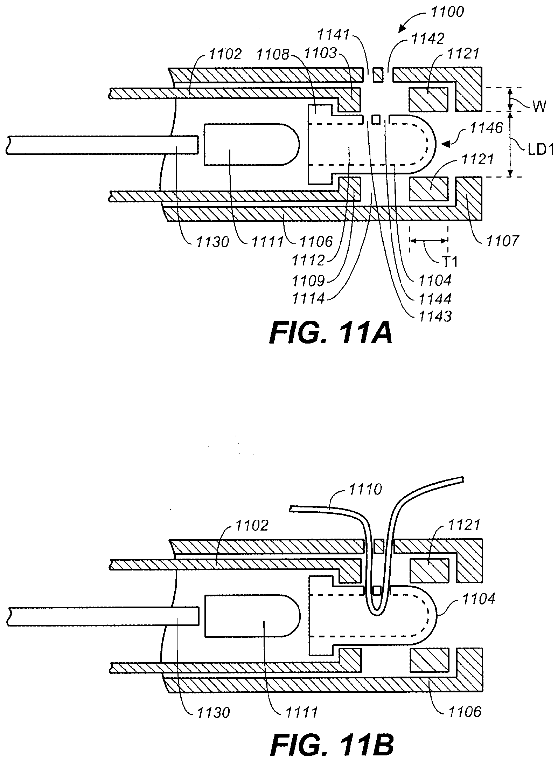

In some variations, a locking device may be configured to secure a tether at multiple (e.g., 2, 3, 4, 5) different locations. For example, FIGS. 11A-11F show a variation of a locking device (1100) comprising a locking body (1104), a plug (1111), and a locking component (1121) (as shown, in the form of a ring, although other suitable configurations may be used). Locking device (1100) may be used to lock one portion of a tether between plug (1111) and locking body (1104), and another portion of the tether between locking body (1104) and locking component (1121), as described in further detail below.

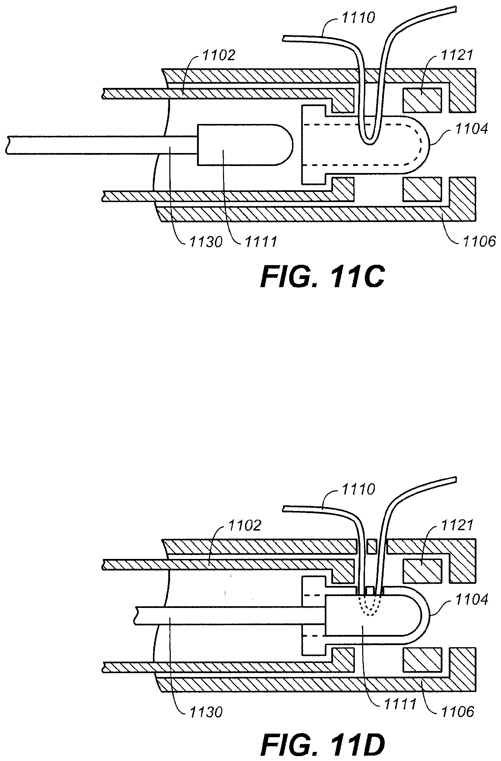

Locking device (1100) further comprises a coupling tube (1102) with a distal portion (1103) releasably coupled to locking body (1104). In this particular variation, locking body (1104) is in the form of a locking tube with a lumen (1112) configured to receive plug (1111), but other variations of locking bodies may have different shapes or configurations. For example, in some variations, a locking body may have a conical shape with a hollow region configured to receive a plug. When plug (1111) is pushed into lumen (1112) of locking body (1104) (e.g., using a pushing member (1130)), a portion of tether (1110) may be compressed between the plug and the wall of the locking body, as illustrated in FIG. 11D. This causes the tether to be secured between the plug and the locking body.

As described above, locking device (1100) comprises a locking component (1121). Locking component (1121) is configured to fit over locking body (1104) and to secure a tether therebetween. This provides locking device (1100) with an additional mechanism for securing a tether, thereby decreasing the likelihood of the tether becoming unsecured. In the variation illustrated in FIGS. 11A-11F, locking device (1100) is capable of securing a tether in at least two locations, with a first location being between locking body (1104) and plug (1111), and a second location between locking component (1121) and locking body (1104). It is contemplated that in other variations, a locking device may secure a tether by using the locking component, but not the plug, or vice-versa. Locking component (1121) may have any of a variety of dimensions and shapes, as discussed in further detail below. Additionally, locking component (1121) may be made of any suitable material or materials, such as, for example, one or more polymers (e.g., nylon or polyether block amide, such as PEBAX® polyether block amide (e.g., PEBAX® 7233)), metals, and/or metal alloys.

As shown in FIG. 11A, locking component (1121) has a thickness (T1) that allows the locking component to cover openings (1143) and (1144) formed in the wall of locking body (1104). Openings (1143) and (1144) allow tether (1110) to pass through locking body (1100), so that the tether may be secured by plug (1111) as the plug enters lumen (1112) of locking body (1104) (e.g., as a result of an interference fit between the plug and the locking body). The dimensions of the locking component and its opening also allow the locking component to fit over the locking body. In certain variations, thickness (T1) of locking component (1121) may be from about 1.27mm (0.05 inch) to about 6.35mm (0.25 inch) (e.g., about 4.8mm (0.19 inch)), width (W) of locking component (1121) may be from about 2.54mm (0.1 inch) to about 2.64mm (0.104 inch), and/or diameter (LD1) of an inner lumen (1146) of locking component (1121) may be about 2mm (0.08 inch).

Although locking component (1121) is configured to fit over a portion of locking body (1104), in some variations, a locking component may be configured to fit over the entirety of a locking body. Moreover, in certain variations, multiple locking components may be employed to fit over a locking body (and, e.g., cover openings in the wall of the locking body). For example, in one variation, a first locking component may be configured to fit over a first opening in a locking body wall, and a second locking body may be configured to fit over a second opening in the locking body wall.

Referring again to FIGS. 11A-11F, locking device (1100) also includes an outer sheath (1106) comprising a lumen (1114) and a shoulder (1107). Sheath (1106) surrounds coupling tube (1102), locking body (1104), and locking component (1121). Sheath (1106) facilitates the coupling of coupling tube (1102) to locking body (1104) by compressing coupling tube (1102) to locking body (1104). As shown, locking body (1104) includes a shoulder (1108), and coupling tube (1102) includes a corresponding shoulder (1109) configured to latch onto locking body shoulder (1108) when sheath (1106) compresses coupling tube (1102) to locking body (1104). Like locking body (1104), sheath (1106) may also comprise openings (1141) and (1142) through which a tether (1110) may pass. While two openings are shown, any suitable number of openings may be used.

Locking device (1100) also includes a pushing member (1130), as described briefly above. Pushing member (1130) may be advanced distally to push plug (1111) into lumen (1112) of locking body (1104), such that shoulder (1108) of locking body (1104) pushes against shoulder (1103) of coupling tube (1102) and shoulder (1107) of sheath (1106). The different variations of the pushing member as described above with reference to the locking device of FIG. 2 may alternatively or additionally be incorporated into locking device (1100) and/or other variations of suitable pushing members may be employed.

As previously described, sheath (1106) and locking body (1104) each comprise two openings (1141) and (1142), and (1143) and (1144), respectively, that allow a tether to pass therethrough. It is contemplated, however, that in other variations, a different number of openings may be formed on the locking body and/or sheath. For example, in some variations, a locking body and/or sheath may include 1, 2, 3, 4, 5, 6, 7, 8, 9, or 10 openings. Moreover, in certain variations, a locking body and/or sheath may not include any openings. In some variations, a sheath and a locking body may include different numbers of openings. For example, in some variations, a sheath may not comprise any openings and a locking body may comprise two openings. In other variations, a sheath may comprise two openings and a locking body may comprise four openings. The openings in a sheath and/or locking body may have any of a variety of cross-sectional shapes. For example, in some variations, an opening may have a circular cross-sectional shape, while in other variations, an opening may have a non-circular cross-sectional shape, such as an oval, rectangular, or other polygonal cross-sectional shape. Moreover, in certain variations, an opening may have a non-polygonal cross-sectional shape, such as an irregular cross-sectional shape.

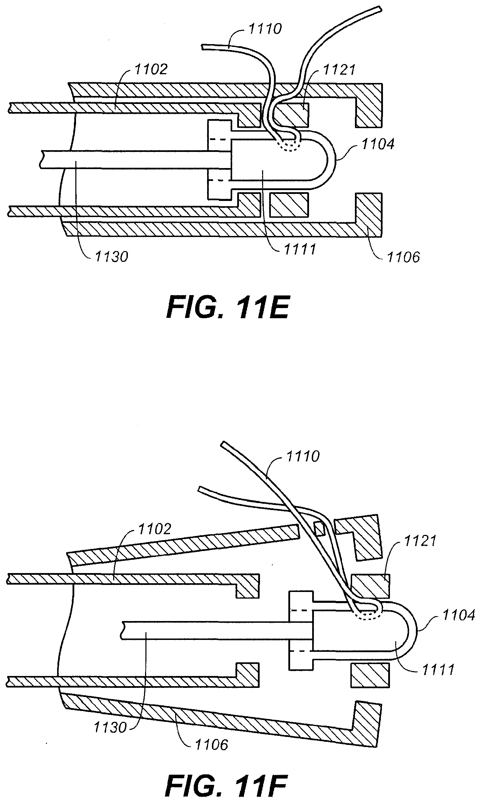

During operation of locking device (1100), tether (1110) may be passed through openings (1141) and (1142) of sheath (1106), and through openings (1143) and (1144) of locking body (1104), as illustrated in FIGS. 11A and 11B. Thereafter, pushing member (1130) may be advanced distally to push plug (1111) toward locking body (1104), as illustrated in FIG. 11C. Next, plug (1111) may be pushed into lumen (1112) of locking body (1106), thereby resulting in a first portion of tether (1110) being compressed between plug (1111) and the wall of locking body (1106), as illustrated in FIG. 11D. Thereafter, pushing member (1130) may be further advanced distally to push locking body (1104) into the inner lumen (1146) of locking component (1121). This allows locking device (1100) to secure a second portion of tether (1110) by compressing the second portion of tether (1110) between locking body (1106) and locking component (1121). Alternatively, instead of configuring locking body (1106) to be pushed into the inner lumen (1146) of locking component (1121), locking component (1121) may be retracted proximally until locking component (1121) fits over locking body (1104) and covers openings (1143) and (1144), as illustrated in FIG. 11E. Next, and as illustrated in FIG. 11F, pushing member (1130) may be further advanced to cause the release of locking body (1104), plug (1111), and locking component (1121) from coupling tube (1102) and sheath (1106). In some variations, the release of these coupled components may be caused by engagement of shoulder (1108) of locking body (1104) with coupling tube shoulder (1109) and sheath shoulder (1107). More specifically, as locking body (1104) is pushed, shoulder (1108) of locking body (1104) eventually presses against coupling tube shoulder (1109). The resulting pressure from such a pushing force, directed at coupling tube shoulder (1109), is configured to cause coupling body (1104) to open, as illustrated in FIG. 11F. Continued pushing of locking body (1104) eventually also causes locking body shoulder (1109) to press against the sheath shoulder (1107). Similar to the mechanism that causes the opening of the coupling tube (1102), the pressure from the pushing force would also eventually cause sheath (1106) to open.

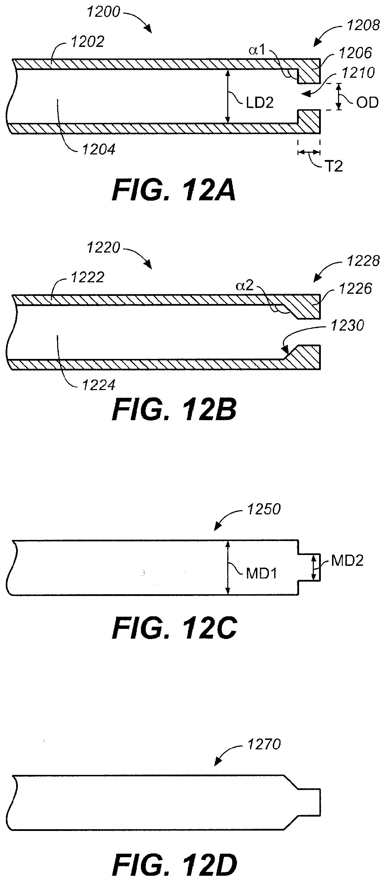

Locking device sheaths may have any of a number of different suitable configurations. For example, FIG. 12A shows a locking device sheath (1200) having a wall portion (1202), a lumen (1204), and a shoulder (1206) at its distal end (1208). Shoulder (1206) is positioned at a right angle (al) with respect to wall portion (1202). Lumen (1204) has a lumen diameter (LD2) that can be, for example, 2.8mm (0.11 inch). Locking device sheath (1200) also includes an opening (1210) at its distal end (1208). Opening (1210) may have a diameter (OD) of, for example, from 2mm (0.08 inch) to 2.16mm (0.085 inch). Shoulder (1206) has a thickness (T2) which may be, for example, from about 0.5 millimeter to about 1.0 millimeter.

Additional variations of locking device sheaths may be employed. For example, FIG. 12B shows a locking device sheath (1220) having a wall portion (1222), a lumen (1224), and a shoulder (1226) at its distal end (1228). Shoulder (1226) is positioned relative to wall portion (1222) at an angle (a2) that is greater than 90°, such that shoulder (1226) has a ramp-shaped section (1230). This ramp-shaped section may, for example, help to promote opening of locking device sheath (1220).

Locking device sheaths may comprise any suitable material or materials, such as one or more polymers (e.g., polyether block amide, such as PEBAX® polyether block amide (e.g., PEBAX® 7233 or PEBAX® 5533)). In some variations, a lumen of a locking device sheath may have a polyimide liner, such as an etched polyimide liner. Alternatively or additionally, the lumen for a coupling line that locks and unlocks an interlocking feature in a locking device sheath may have a polyimide liner. In certain variations, a mandrel may be used to form the coupling line lumen, and the mandrel may be formed of, for example, polytetrafluoroethylene-coated stainless steel. In certain variations, a locking device sheath may comprise different portions that are made of different materials. Moreover, a locking device sheath may comprise wall portions including braiding, and/or wall portions that do not include braiding. In some variations, a locking device sheath may comprise a hypotube (e.g., a stainless steel hypotube) and/or one or more radiopaque markers (e.g., platinum markers).

FIGS. 12C and 12D show different variations of mandrels that may be used to form locking device sheaths. FIG. 12C shows a mandrel (1250) that may be used, for example, to form a locking device sheath having a shoulder that extends at a right angle with respect to a wall portion of the locking device sheath (e.g., such as locking device sheath (1200) of FIG. 12A). Mandrel (1250) has a first larger diameter (MD1) and a second smaller diameter (MD2). In some variations, MD1 may be about 2.8mm (0.11 inch). Alternatively or additionally, MD2 may be from about 2mm (0.08 inch) to about 2.16mm (0.085 inch). FIG. 12D shows a mandrel (1270) that may be used, for example, to form a locking device sheath having a shoulder that extends at an angle of greater than 90° with respect to a wall portion of the locking device sheath (e.g., such as locking device sheath (1220) of FIG. 12B).

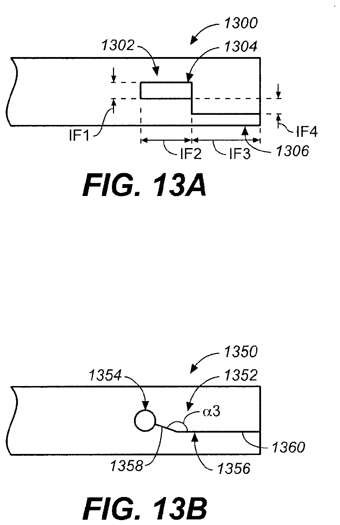

FIGS. 13A and 13B show locking device sheaths comprising interlocking features that may be locked and unlocked using a coupling line, such as a wire (not shown). FIG. 13A shows a locking device sheath (1300) having an interlocking feature (1302) formed of a rectangular opening (1304) and a slit portion (1306). Rectangular opening (1304) has dimensions (IF1) and (IF2), while slit portion (1306) has dimensions (IF3) and (IF4). FIG. 13B shows a locking device sheath (1350), which has an interlocking feature (1352) formed of a circular opening (1354) and a slit portion (1356). Slit portion (1356) comprises two sections (1358) and (1360) that form an angle (a3) with respect to each other. Angle (a3) is greater than 90°. The size of angle (a3) may help to promote the opening of locking device sheath (1350) (e.g., when the locking device sheath comprises a shoulder, as described above). For example, the size of angle (a3) may help to reduce the likelihood of interference between the portions of interlocking feature (1352) on either side of slit portion (1356), as the interlocking feature is unlocked and the portions part away from each other.

While locking device sheaths with certain interlocking figure configurations have been shown, it should be understood that any other suitable configurations may be employed for a locking device sheath. As an example, a locking device sheath may include multiple interlocking features, such as 2, 3, 4, or 5 interlocking features. The interlocking features may, for example, be radially spaced along the circumference of the locking device sheath. They may be separated from each other by the same distance or by different distances, and may have the same configuration or different configurations. Moreover, in certain variations, an interlocking feature may include one or more openings that are not rectangular or circular. For example, an interlocking feature may include an oval opening (e.g., with its longest dimension being 2.43mm (0.096 inch) and its shortest dimension being 1mm (0.04 inch).

The lumen for the coupling line that is used to lock and unlock interlocking feature (1302) may be located along the longitudinal center line of locking device sheath (1200), or above or below the longitudinal center line (e.g., by about 0.25mm (0.01 inch)). Varying the location of a coupling line lumen may, for example, allow for a different interlocking feature configuration to be employed in a locking device sheath.

As shown above, an interlocking feature may be formed of at least two slits that are angled with respect to each other. The location of this angle may be close to (e.g., about 1 millimeter) or far from (e.g., from about 2 to about 3 millimeters) the lumen for the coupling line that locks and unlocks the interlocking feature. In some variations, as the location of the angle becomes more distant from the coupling line lumen, the locking device sheath may be easier to open (e.g., when withdrawing the coupling line). Moreover, a locking device sheath having an interlocking feature with a relatively long overall length may be easier to open than a locking device sheath having an interlocking feature with a relatively short overall length.

The diameter of a coupling line that is used to lock and unlock an interlocking feature of a locking device comprising a locking body and a locking component may be selected to be large enough to limit or prevent kinking when the locking component is positioned over the locking body. In some variations, the diameter of the coupling line may be from about 2.3mm (0.09 inch) to about 0.28mm (0.011 inch).

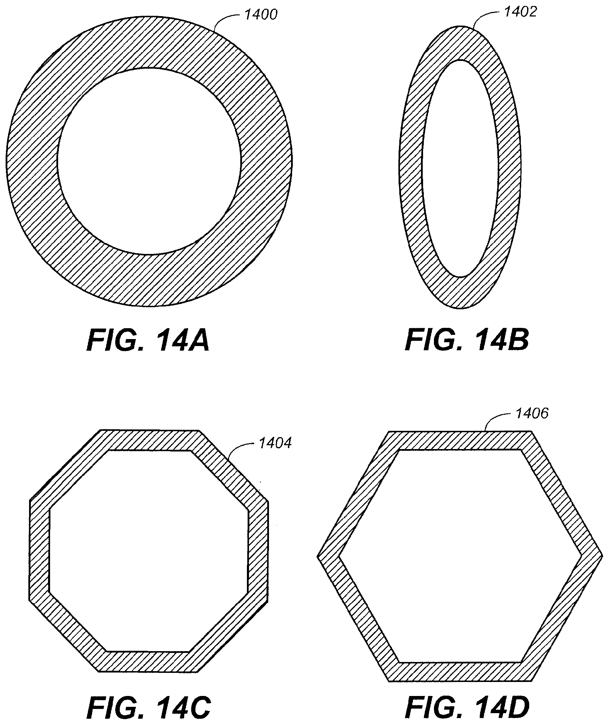

As discussed above, a locking component may have any of a variety of cross-sectional shapes. For example, as illustrated in FIG. 14A, a locking component (1400) may have a substantially circular cross-sectional shape. In other variations, a locking component may have a non-circular cross sectional shape. For example, FIG. 14B shows a locking component (1402) having an oval shape, while FIGS. 14C and 14D show locking components having polygonal shapes (e.g., octagonal locking component (1404) in FIG. 14C and hexagonal locking component (1406) in FIG. 14D).

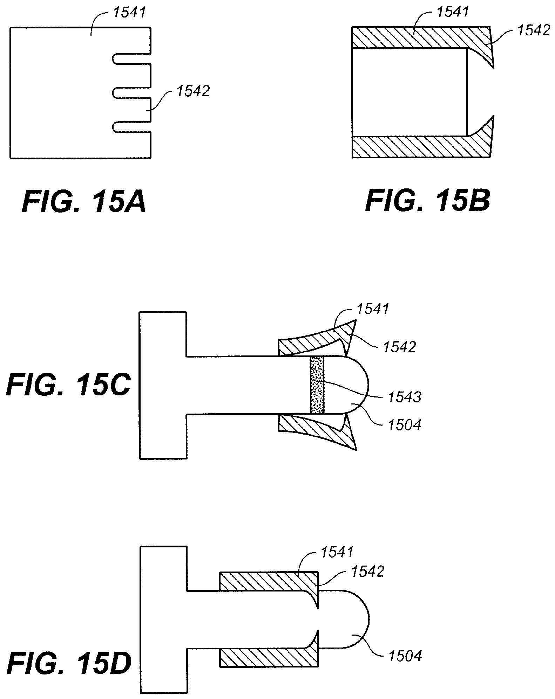

In some variations, a locking component may comprise one or more structures to facilitate the securing of the locking component to a locking body. These securing structures may include, but are not limited to, surface texturing, ribs, and/or grooves. As an example, FIGS. 15A and 15B show a locking component (1541) comprising shoulders (1542) on its distal end that allow for radial expansion of the locking component at its distal end. Shoulders (1542) are configured to mate with a groove (1543) on a locking body (1504), as shown in FIGS. 15C and 15D. Locking component (1541) may be changed from an unlocked configuration (as shown in FIG. 15C) to a locked configuration (as shown in FIG. 15D). In some variations, locking may be achieved by distal advancement of the locking body and/or proximal retraction of the locking component.

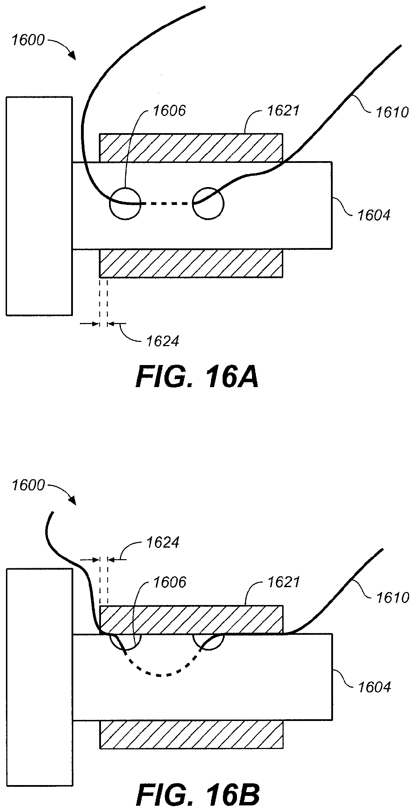

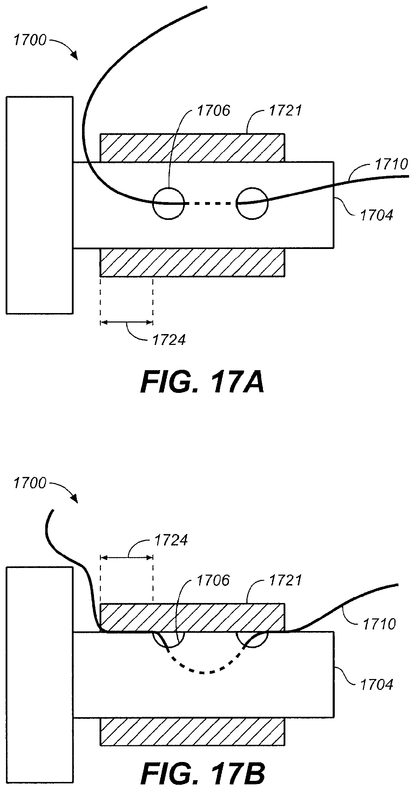

Referring to FIGS. 16A, 16B, 17A and 17B, different locking body variations having openings at different positions are shown. FIGS. 16A and 16B show top and side views, respectively, of one variation of a locking device (1600) comprising a locking component (1621) and a locking body (1604) securing a tether (1610) therebetween. As shown there, a distance (1624) between the edge of a proximal opening (1606) in locking body (1604) and the edge of locking component (1621) when the locking body is fitted over the locking body is relatively short. By contrast, FIGS. 17A and 17B show top and side views, respectively, of a locking device (1700) comprising a locking component (1721) and a locking body (1704) securing a tether (1710) therebetween. As shown there, a distance (1724) between the edge of a proximal opening (1706) in locking body (1704) and the edge of locking component (1721) when the locking body is fitted over the locking body is relatively long. The distance between the edge of a proximal opening in a locking body and the edge of a locking component may affect the degree of security with which locking component secures a tether against locking body. In some instances, a greater distance may correspond to an increased level of tether security relative to the tether security achieved with a lesser distance. This may be because the tether is less likely to slip out from between the locking body and the locking component as the distance increases (e.g., because a greater length of tether is captured between the locking body and the locking component). A greater distance may also correspond with a greater force required to secure the tether. In certain variations, the distance between the edge of a proximal opening in a locking body and the edge of a locking component may be from about 1mm (0.04 inch) to about 5mm (0.2 inch).

Although only a few of the ways in which a locking element may be releasably coupled to a device have been described, it should be understood that any appropriate coupling may be used, including snap fits and other coupling mechanisms (e.g., threads, etc.). Additionally, the couplings described herein may be readily scaled in size for use even with applications that may require very small locking elements (e.g., for use in percutaneous applications and/or surgical applications, such as microsurgical applications). Locking elements that are releasably coupled to devices are described, for example, in U.S. Patent Application Publication No. US 2008/0172035 A1.

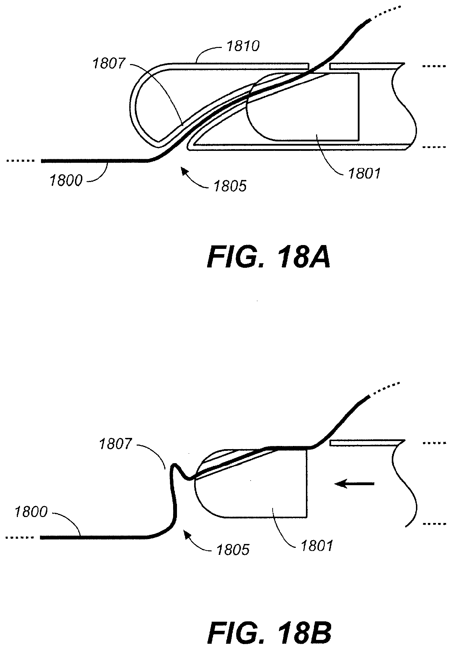

While certain variations of locking devices have been described above, any appropriate locking device may be used to secure a tether. For example, a locking device may comprise a kinking tube that is kinked to secure a tether. As an example, FIGS. 18A and 18B show one variation of a locking device (1805) having a locking element that fixes a tether in a tight winding path to secure the tether. More specifically, locking device (1805) includes an inner tube (1807) that is capable of being kinked (to thereby secure the tether) and un-kinked (to thereby release the tether). In FIG. 18A, inner tube (1807) is un-kinked, allowing a tether (1800) to pass freely though the locking element (e.g., outer tube (1810)). In FIG. 7B, inner tube (1807) has been kinked, so that tether (1800) is constrained, and cannot slide freely within inner tube (1807). Additionally, in FIG. 18B, a plug (1801) is pushed forward into the distal end of the locking element, compressing inner tube (1807) and further securing tether (1800) into the locked position.

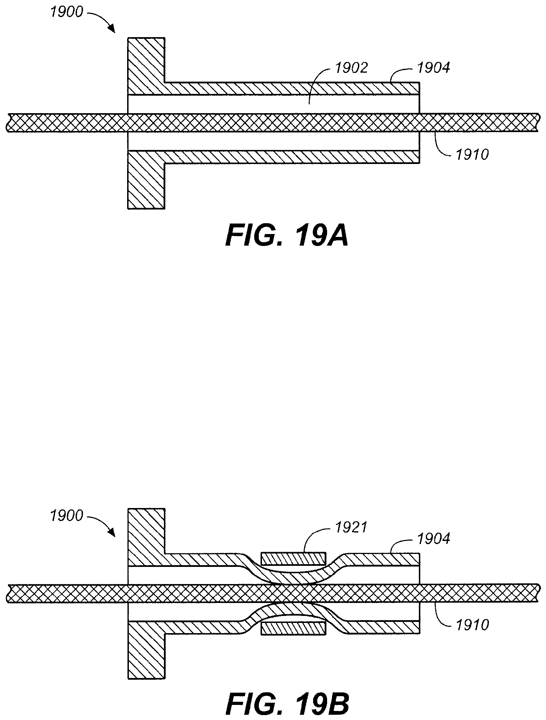

FIGS. 19A and 19B show another variation of a locking device (1900). As shown, locking device (1900) comprises a locking body (1904) and a locking component (1921). In this particular variation, a tether (e.g., tether (1910)) may be routed through the entire length of a lumen (1902) of locking body (1904). Locking component (1921) is then fitted over a portion of locking body (1904). Although locking component (1921) is depicted in FIG. 19B as substantially fitting over the middle section of locking body (1904), it is contemplated that locking component (1921) may also be located at other suitable positions. When locking component (1921) is fitted over locking body (1904), it compresses locking body (1904). This action, in turn, causes the inner wall of locking body (1904) to compress tether (1910). This compression causes tether (1910) to be secured within the locking device.

In some methods, it may be necessary to load a tether into a device, such as a locking device, a cutting device, or a combination locking and cutting device. Various methods and/or devices may be used to accomplish this loading.

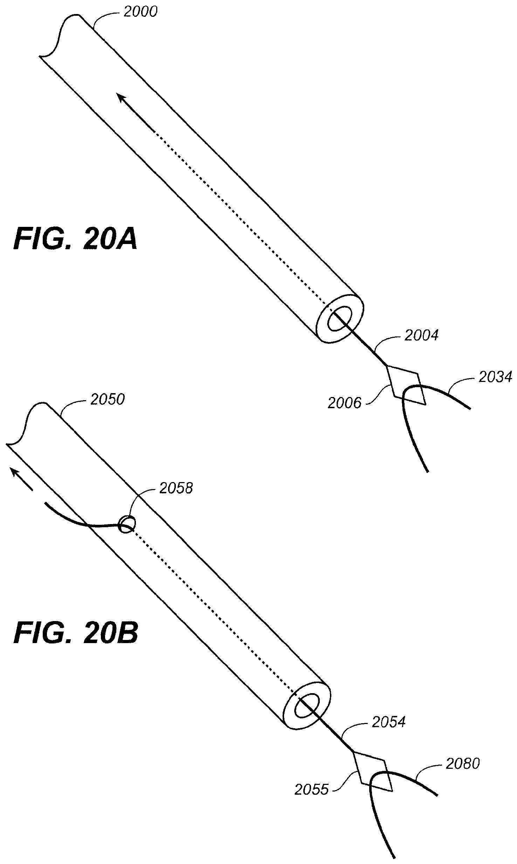

As an example, and referring now to FIGS. 20A and 20B, in some variations, a tether (2034) is loaded into a device (2000) using a lasso (2004) which comprises a loop (2006) at one end. One end of tether (2034) is threaded through loop (2006) of lasso (2004). Lasso (2004) may then be pulled along the longitudinal axis of device (2000) (FIG. 20A), to load tether (2034) into device (2000). In alternative implementations, shown in FIG. 20B, a lasso (2054) having a loop (2055) may be pulled through a side hole (2058) in a device (2050) to load a tether (2080) into the device. Device (2000) or device (2050) may be used to perform one or more functions, such as locking and/or cutting (described in further detail below). Lassos may be made from, for example, conventional materials such as wire, suture, cable, string, or a monofilament. A lasso may comprise a loop (as show in FIGS. 20A and 20B), a hook, a coil, a tube, an elongate element with a hole, or any other structure or material that can "grab" a tether.

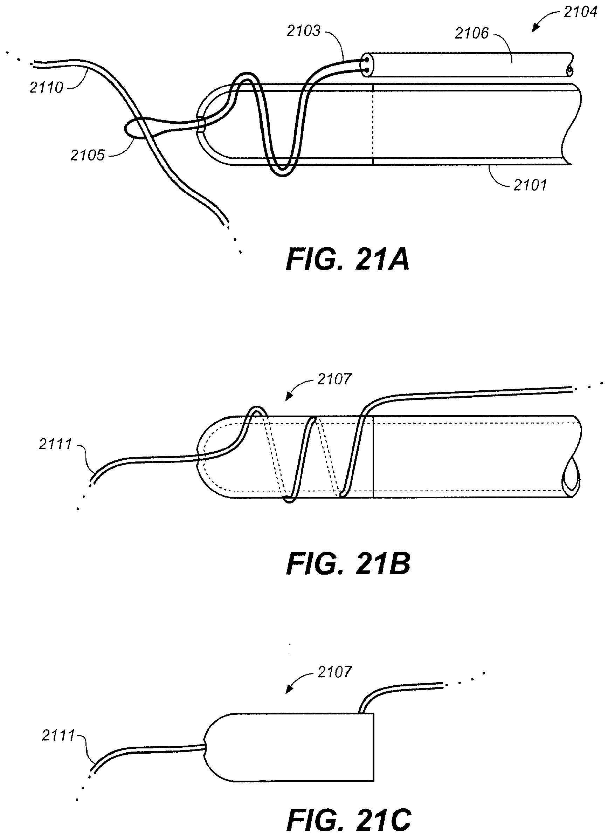

FIG. 21A shows a variation of a tether-loading device (2104) that may be used to load a tether into another device. As shown in FIG. 21A, tether-loading device (2104) is preloaded into a locking device (2101). Tether-loading device (2104) comprises a rod-shaped member (2106) and a wire (2103) extending from an end of the rod-shaped member. Wire (2103) forms a loop (2105) (e.g., a lasso), and the flattened loop passes through holes (or passages) in the locking device. A tether (2110) is passed through the loop, and drawn into the locking device, as previously described. While the use of tether-loading devices to load tethers into locking devices has been described, such tether-loading devices may have other uses, such as to load tethers into cutting devices or combination locking and cutting devices (described in further detail below). Other uses may also apply.

In some variations, a locking device may include channels, guides, or passages which direct the tether. For example, FIG. 21B shows a portion of a device having a detachable locking element (2107). The device includes passages and guides which can be used to position a tether (2111) within the device when the tether is coupled to the device. The tether may be held so that it can be secured, and then cut, using the device. FIG. 21C shows an example of detachable locking element (2107), in which locking element (2107) has been secured to tether (2111) and released from the rest of the device (e.g., as described above with reference to FIG. 2). Any of the features described herein with respect to a locking device may also be used, as appropriate, in a cutting device, or in a combined locking and cutting device.

While the use of tether-loading devices to load tethers into locking devices has been described, such tether-loading devices may have other uses, such as to load tethers into cutting devices or combination locking and cutting devices (described in further detail below). Other uses may also apply. Moreover, any of the features described herein with respect to a locking device may also be used, as appropriate, in a cutting device, or in a combined locking and cutting device.

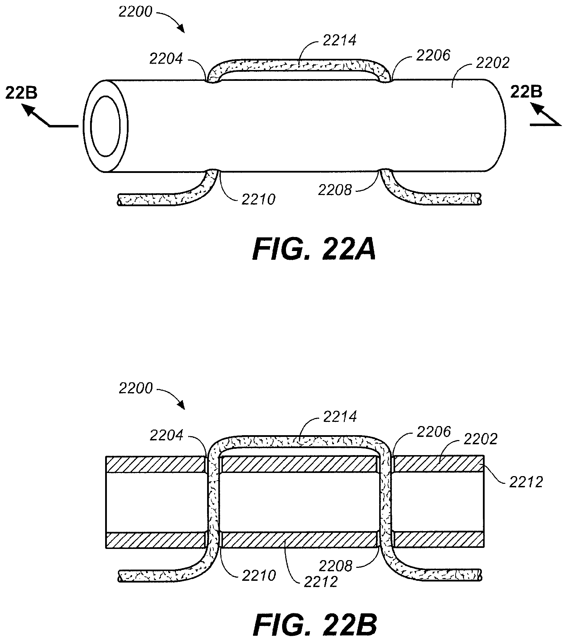

Tethers can be routed through a device, such as a locking device or a cutting device, in any of a number of different configurations. For example, FIGS. 22A and 22B show a variation of a locking device (as shown, a locking catheter (2200)). Locking catheter (2200) includes a tubular member (2202) having a wall (2212) with four openings (2204), (2206), (2208), and (2210) formed in it. A locking catheter such as locking catheter (2200) may be used, for example, to maintain tension in a tether, and to stabilize the tether for cutting. In FIGS. 22A and 22B, a tether (2214) has been threaded into locking catheter (2200), through openings (2204), (2206), (2208), and (2210). The tether may be threaded into the locking catheter using, for example, a lasso, such as one of the lassos described above. The lasso may have a relatively flexible loop which may enhance the maneuverability of the lasso through the openings in the locking catheter.

While locking catheter (2200) is shown as including four openings through which tether (2214) is threaded, locking catheters can include other numbers of openings. For example, some variations of locking catheters may include fewer openings (e.g., two openings), while other variations of locking catheters may include more openings (e.g., six openings, eight openings, etc.). As the number of openings in a locking catheter increases, the likelihood of movement by a tether that is threaded through the openings may decrease.

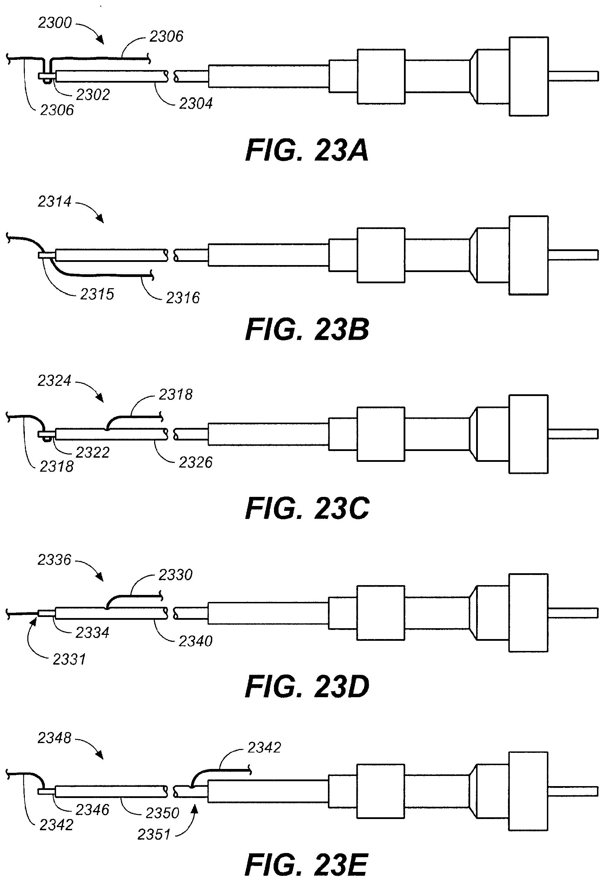

Additional non-limiting examples of routing configurations for tethers are shown in FIGS. 23A-23E. Referring to FIG. 23A, a locking catheter (2300) includes a tip (2302) and a shaft (2304). A tether (2306) is threaded through four openings in the tip, so that the locking catheter secures the tether, thereby maintaining the tension in the tether. While tether (2306) is threaded through four openings in tip (2302), tethers can be threaded through different numbers of openings in a catheter tip. For example, FIG. 23B shows a locking catheter (2314) including a tip (2315), and a tether (2316) that has been threaded through two openings in the tip of the locking catheter. In FIG. 23C, a tether (2318) is threaded through three openings in a tip (2322) of a locking catheter (2324), and also is threaded into the shaft (2326) of the locking catheter. The tether eventually exits the shaft through an opening in the shaft. FIG. 23D shows a tether (2330) that is threaded into the distal end (2331) of the tip (2334) of a locking catheter (2336), and that extends through the shaft (2340) of the locking catheter, exiting through an opening in the shaft. Tethers can extend through varying lengths of a locking catheter shaft. For example, the tether shown in FIG. 23D extends through a shorter catheter shaft length than does the tether shown in FIG. 23E. More specifically, tether (2330) of FIG. 23D extends through only a distal portion of shaft (2340) of locking catheter (2336). By contrast, FIG. 23E shows a tether (2342) that is threaded into an opening in a tip (2346) of a locking catheter (2348), and that extends through almost the entire length of the shaft (2350) of the locking catheter. Tether (2342) exits the shaft through an opening in the proximal end (2351) of the shaft.

As described above, in operation, a locking element may be secured to a tether to fix the length of the tether and/or to prevent the tether from moving. After the tether has been locked, any excess length of the tether may be cut and removed. In some variations in which a detachable locking element is used, a tether may be cut to remove excess material either before or after detaching the locking element from the rest of the device. Generally, the tether is cut proximal to the locking mechanism. In many cases, it may be desirable to cut the tether as closely as possible to the locking mechanism, while leaving enough excess length to allow for any slippage that may occur. Examples of various methods and devices that may be used to cut excess tether are described in more detail below.

In some variations, a cutting device comprising two or more concentric tubes can be used to cut excess tether. For example a cutting device may comprise two concentric tubes, and during use, one concentric tube may be advanced relative to another concentric tube to shear off excess tether at a desired position. Alternatively or additionally, one concentric tube may be rotated with respect to another concentric tube to cut the tether.

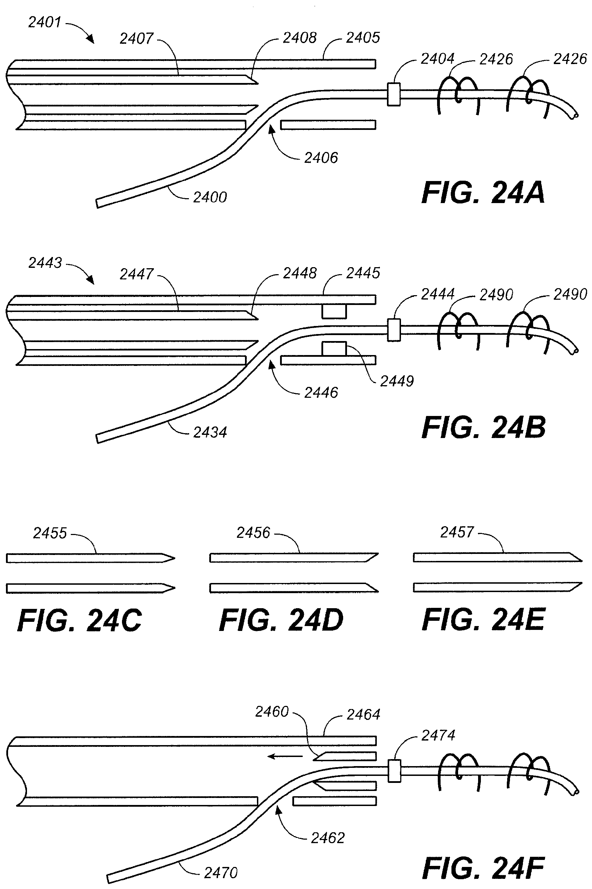

FIG. 24A shows a cutting device (2401) that may be used to cut a tether (2400) extending through anchors (2426). Cutting device (2401) comprises a catheter (2405) and a tubular cutter (2407) disposed within catheter (2405). As shown in FIG. 24A, tether (2400) has been fixed by a locking element (2404), and has been threaded into catheter (2405) such that it exits through a side opening (2406) in the catheter. Tether (2400) can be threaded into catheter (2405) by any suitable method including, for example, one or more of the methods described above. Tubular cutter (2407) has an edge (2408) that is sufficiently sharp to cut a tether. For example, tubular cutter (2407) may be in the form of a metal tube having a sharpened edge. During use, tubular cutter (2407), which is attached to a flexible tube or a rod, is advanced within catheter (2405) such that the tubular cutter passes over side opening (2406). As tubular cutter (2407) is advanced over tether (2400), tubular cutter (2407) shears off the excess portion of the tether. While tubular cutter (2407) is tubular in shape, other configurations of cutters may be used. For example, a cutter may be semitubular (e.g., having a shape similar to a half-pipe), or may have any other suitable configuration. In some variations, a cutter may not be tubular or semitubular. As an example, a cutter may be in the form of a flat blade.

In some variations, and as shown in FIG. 24B, a cutting device (2443) comprises a catheter (2445), a base (2449) positioned on an interior surface of the catheter, and a tubular cutter (2447) concentrically disposed within the catheter. While cutter (2447) is tubular, other configurations of cutters may be used, as described above. Base (2449) can, for example, be in the form of a block that is attached to the interior surface of catheter (2445), or that is integral with the interior surface of catheter (2445). Base (2449) can be formed of any suitable material, such as any elastomeric or rigid material. FIG. 24B shows cutting device (2443) being used to cut a tether (2434) extending through anchors (2490), into catheter (2445), and through a side opening (2446) in catheter (2445). Prior to being cut, tether (2434) is fixed in place by a locking element (2444). Then, tubular cutter (2447) is advanced to cut tether (2434). Tubular cutter (2447) is advanced against base (2449), which assists tubular cutter (2447) in cutting tether (2434). In some variations, tubular cutter (2447) can be spun or rotated to improve cutting.

Tubular cutters can have any suitable cutting edge configuration. For example, a tubular cutter may have a beveled cutting edge, as exemplified by tubular cutter (2455) of FIG. 24C, a sharpened outer cutting edge, as exemplified by tubular cutter (2456) of FIG. 24D, or a sharpened inner cutting edge, as exemplified by tubular cutter (2457) of FIG. 24E. In addition, a tubular cutter may have a serrated or saw-tooth pattern of sharp protrusions around its perimeter to aid in cutting. Such variations may be used, for example, when the tubular cutter is spun or rotated during the cutting process.

In some variations, and as shown in FIG. 24F, a tubular cutter (2460) can be positioned in front of a side opening (2462) in a catheter (2464). Tubular cutter (2460) can then be pulled in a proximal direction toward side opening (2462) (indicated by solid arrow) to cut a tether (2470) extending through side opening (2462), which has been fixed by a locking element (2474). Pulling a cutter proximally may provide for a relatively easy and/or efficient way of cutting a tether.

FIG. 25 shows another variation of a cutting device in which a cutter is translated proximally to cut a tether. As shown there, a cutting device (2500) comprises a tubular elongated member (2502) having a lumen (2503), and a cutter (2504) disposed within the lumen of the elongated member. Cutter (2504) has a cutting blade (2506) that faces in a proximal direction. Elongated member (2502) comprises a wall portion (2508) having two openings (2510) and (2512) through which a tether (2514) is threaded, such that the tether crosses the lumen of the elongated member. While two wall portion openings are shown, other variations of devices may include a different number of wall portion openings, such as three or four wall portion openings. When it is desired to sever tether (2514), cutter (2504) is pulled proximally using a pulling member (2516) that is attached to cutter (2504). This causes cutting blade (2506) to contact and sever tether (2514). While cutter (2504) is pulled proximally using pulling member (2516), in some variations, a cutter disposed within the lumen of an elongated member may alternatively or additionally be pushed in a proximal direction. For example, a pushing member may be placed into the elongated member at its distal end, and used to push the cutter toward the proximal end of the elongated member.

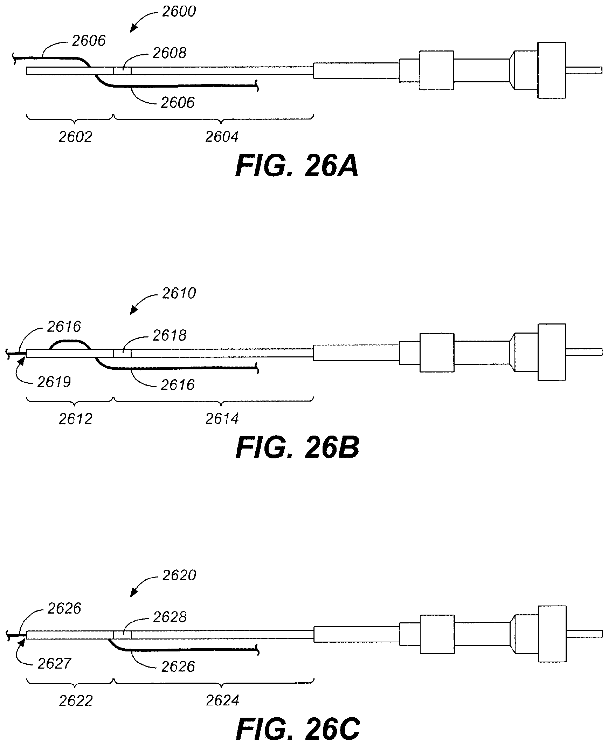

A tether can be threaded through a cutting device in any of a number of different ways using, for example, one or more of the methods described above (e.g., using a lasso). FIGS. 26A-26C show various non-limiting examples of different routing configurations of tethers through cutting catheters. FIG. 26A shows a cutting catheter (2600) including a tip portion (2602) and a shaft portion (2604). A tether (2606) is threaded through two openings in tip portion (2602). A cutter (2608) is disposed within a lumen of shaft portion (2604), and can be used to cut the tether. FIG. 26B shows a different routing configuration for a tether that is threaded through a cutting catheter. As shown in FIG. 26B, a cutting catheter (2610) includes a tip portion (2612) and a shaft portion (2614) containing a cutter (2618). A tether (2616) is threaded into tip portion (2612) at its distal end (2619), and through three openings in the tip portion. Referring now to FIG. 26C, a cutting catheter (2620) includes a tip portion (2622) and a shaft portion (2624). A tether (2626) is threaded into the tip portion (2622) at its distal end (2627), and exits the tip portion through an opening that is proximal to distal end (2627). A cutter (2628) is disposed within shaft portion (2624). Tethers can be threaded through different numbers of holes in cutting catheters and/or through different locations in cutting catheters. All of the described threading variations are merely illustrative examples of suitable threading techniques. Furthermore, other routing configurations may be used to thread a tether through a catheter. As an example, in some variations, a tether may not be threaded through a tip of a catheter.

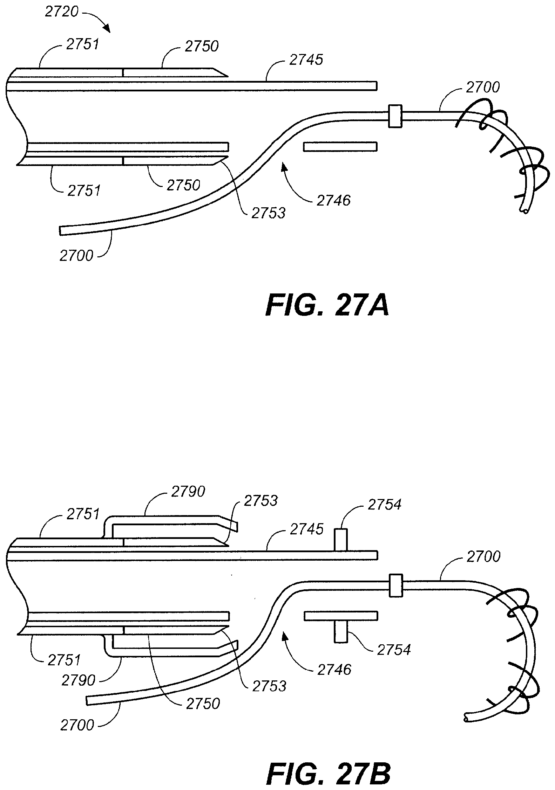

The cutting devices shown above in FIGS. 24A-24F, 25, and 26A-26C comprise cutters that are located internally of their catheters. However, some variations of cutting devices include a catheter and one or more cutters that are located externally of the catheter. For example, as shown in FIG. 27A, a cutting device (2720) includes a catheter (2745) and a tubular cutter (2750) that is configured to slide along the exterior of catheter (2745). Tubular cutter (2750) can, for example, be in the form of a sharpened metal tube (e.g., having a beveled edge). In some variations, and as shown, tubular cutter (2750) is attached to a second tube (2751) which also is configured to slide along the exterior of catheter (2745). In certain variations, second tube (2751) can be flexible.

During use of cutting device (2720), a tether (2700) may be threaded into catheter (2745), and may exit catheter (2745) through a side opening (2746). Tether (2700) can be threaded into catheter (2745) using any suitable method, including methods described herein. As tubular cutter (2750) is advanced in a distal direction toward side opening (2746), end (2753) of tubular cutter (2750) severs tether (2700).

As shown in FIG. 27B, in some variations, a base (2754) can be positioned along catheter (2745), to assist in the tether-cutting process. During use, tether (2700) may be pushed against base (2754) as tubular cutter (2750) is advanced toward side opening (2746). In certain variations, and as also shown in FIG. 27B, a cover or shroud (2790) can be provided around tubular cutter (2750) to limit the likelihood of sharpened end (2753) catching on tissue or the like. In some variations, cover (2790) is attached to second tube (2751).

In certain variations, a cutting device, such as a cutting catheter, may include one or more guides or guards that are configured to prevent a cutter in the cutting device from cutting a wall of the cutting device. A guide may help to direct a cutter in an appropriate direction in or on a device, and a guard may help to shield a cutter from other components of a device.

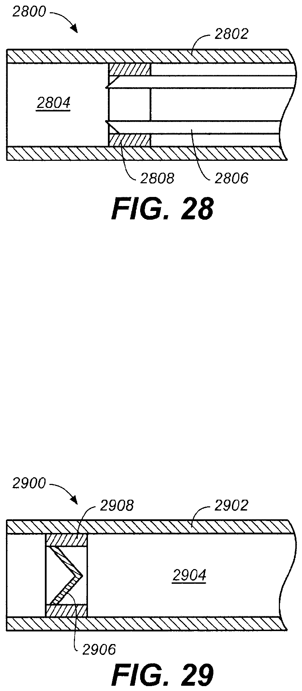

For example, FIG. 28 shows a cutting device (2800) comprising a tubular elongated member (2802) having a lumen (2804), and a cutter (2806) disposed within the lumen. Cutter (2806) is surrounded by a guard (2808) that helps to limit the likelihood of the cutter contacting the wall of elongated member (2802) and causing damage. Any appropriate configuration and combination of cutter and guard or guide may be used. For example, FIG. 29 shows a cutting device (2900) comprising a tubular elongated member (2902) having a lumen (2904), and a V-shaped cutter (2906) disposed within the lumen. The V-shaped cutter is surrounded by a guard (2908) that protects the wall of the elongated member from the cutter. While FIG. 29 shows V-shaped cutter (2906) being completely surrounded by guard (2908), some variations of devices may comprise a V-shaped cutter (or a cutter of a different shape) that is only partially surrounded by a guard.

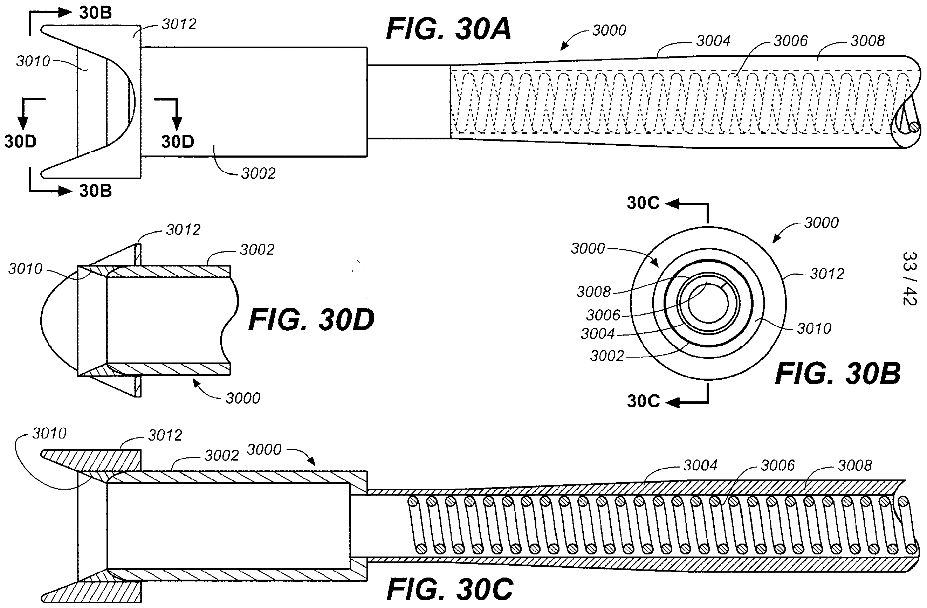

FIGS. 30A-30C show another variation of a cutting device comprising a cutter guard. As shown there, a cutting device (3000) includes a cutter (3002) attached to an elongated member (3004) comprising a coil (3006) surrounded by a support tube (3008). The elongated member may be used to push and/or pull the cutter as desired. It should be noted that certain device variations may comprise an elongated member that is attached to a cutter but that has a different configuration from the elongated member shown in FIGS. 30A- 30C (e.g., an elongated member that does not comprise a coil, or that comprises a coil that is not surrounded by a support tube). Cutter (3002) includes a cutting blade (3010) that is surrounded by a guard (3012) having a U-shaped cross- section. Guard (3012) is shaped in such a way as to direct a tether toward the cutting blade when the tether comes into contact with the guard. At the same time, by shielding the cutting blade the guard protects other components of the cutting device from the cutter. The guard may also help to protect the cutting blade from other components of the cutting device (e.g., maintaining the sharpness of the cutting blade).

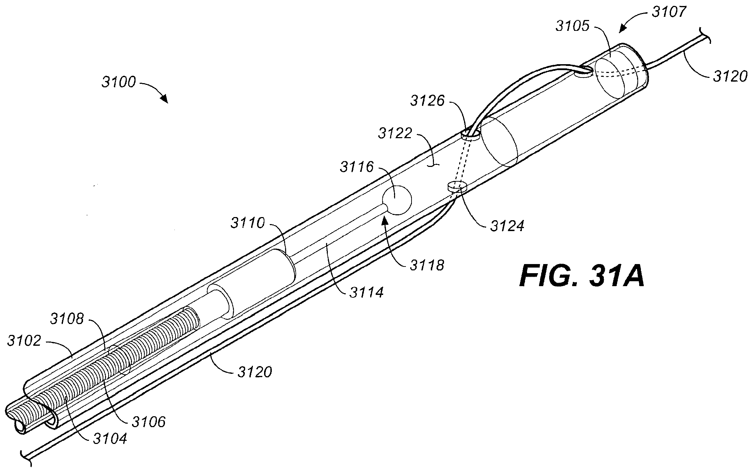

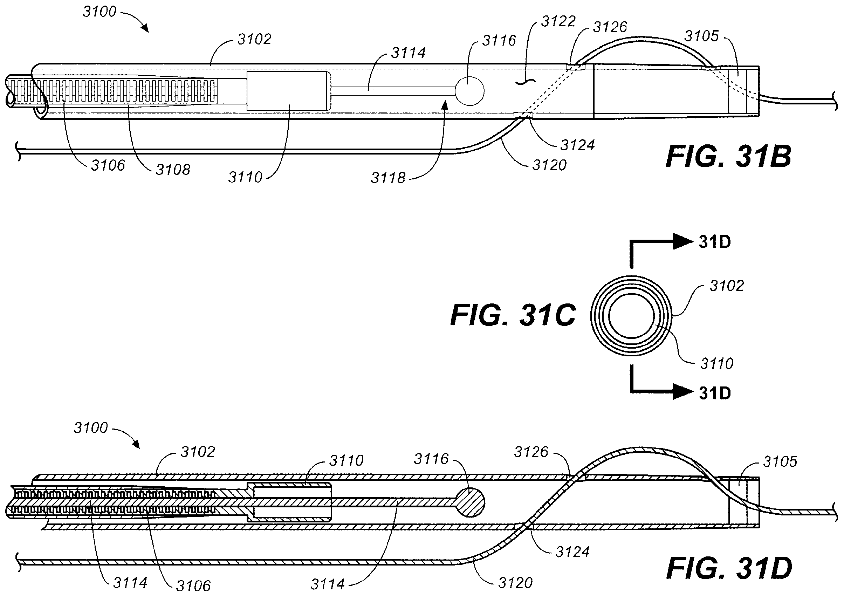

FIGS. 31A-31D show a cutting catheter (3100) that may be used to cut a tether. As shown there, cutting catheter (3100) includes an outer catheter jacket (3102) and a pushing member (3104) contained within the outer catheter jacket. A radiopaque marker band (3105) is located at the distal end (3107) of outer catheter jacket (3102). While one marker band is shown, multiple marker bands or no marker bands may be used in other variations. Furthermore, in some variations, other types of radiopaque markers may alternatively or additionally be used. Moreover, marker bands may be located in any suitable position along a cutting and/or locking device.

Pushing member (3104) includes a coil (3106) surrounded by a support tube (3108), a cutter (3110) located distally of the support tube, and a wire (3114) that extends through the center of coil (3106) and out through cutter (3110). A guide (3116) is formed at the distal end (3118) of wire (3114). As shown, a tether (3120) is threaded into a lumen (3122) of cutting catheter (3100), entering the lumen through a first opening (3124) in the wall of outer catheter jacket (3102), and exiting the lumen through a second opening (3126) in the wall of outer catheter jacket (3102). Tether (3120) forms a diagonal path through lumen (3122), although other configurations may be used. For example, in some variations, a tether may form a path that is substantially perpendicular to a longitudinal axis of a cutting catheter.

During use of the cutting catheter, pushing member (3104) is pushed distally (in the direction of marker band (3105)), until cutter (3110) severs tether (3120). The diagonal positioning of tether (3120) within lumen (3122) may reduce the likelihood of the tether being severed in more than one location. Once the tether is severed, it loses its tension, and thus may not be sufficiently taut to result in a second region of the tether being severed when cutter (3110) comes into contact with it. The severing of the tether in only one location can, for example, limit or prevent the formation of small, free-floating pieces of tether within the body.

Guide (3116) functions to help navigate the cutter through lumen (3122), and to limit the likelihood of cutter (3110) accidentally contacting and cutting outer catheter jacket (3102). Guide (3116) is configured such that during use, the guide contacts the walls of the outer catheter jacket, steering the cutter away from the walls, and thereby limiting contact between the cutter and the walls. For example, during use, the cutting catheter may become curved in one or more locations (e.g., if the cutting catheter is navigated through a tortuous area). As pushing member (3104) is pushed distally, guide (3116), which is located distally of all of the other components of pushing member (3104), comes into contact with the walls of outer catheter jacket (3102) in the curved region or regions. Thus guide (3116) comes into contact with the outer catheter jacket walls before cutter (3110) can. Guide (3116) maintains contact with the walls of outer catheter jacket (3102) throughout the curved region or regions, effectively pushing cutter (3110) away from the walls. By keeping cutter (3110) away from the walls, guide (3116) can prevent cutter (3110) from cutting the walls.

While guide (3116) in FIG. 31A is spherical as shown, any other appropriate shape may be used for a guide, including but not limited to teardrop, ellipsoid, curl, or coil shapes. In some variations, a guide may have a relatively smooth surface over which a tether can easily pass. The relatively smooth surface may also help to maintain the overall profile of the device (e.g., because the relatively smooth surface lacks protrusions that could disrupt the overall profile of the device). Additionally, multiple guides may be used in conjunction with each other. The guides may be of the same shape and/or size, or may be of different shapes and/or sizes. A guide such as guide (3116) may be formed, for example, of one or more metals, metal alloys, and/or plastics. In certain variations, a guide may be formed separately from a wire that is to be used in a pushing member, and may subsequently be attached (e.g., welded or adhesive-bonded) to the wire. In other variations, a guide may be integral with a wire that is to be used in a pushing member.

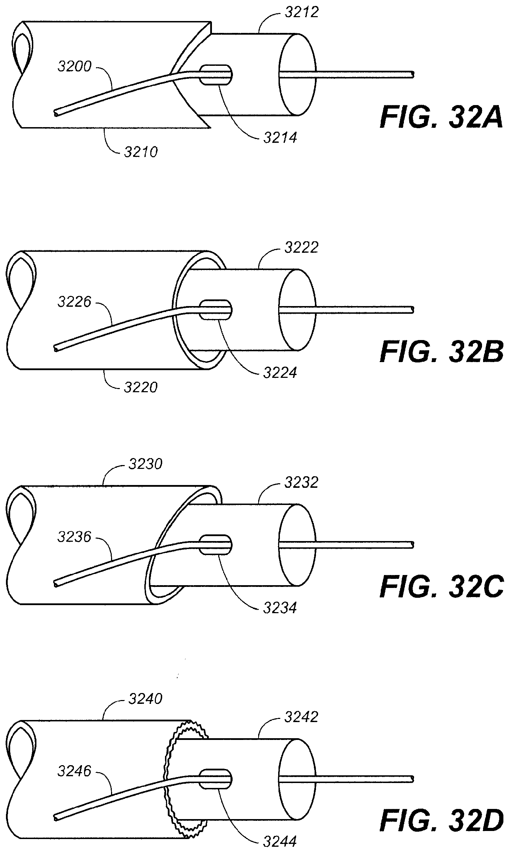

While tubular cutters having certain configurations have been shown, a tubular cutter can have any suitable configuration. For example, as shown in FIG. 32A, a tubular cutter (3210) has a V-shaped cutting edge designed to channel a tether, such as tether (3200). Tubular cutter (3210) is externally disposed relative to a catheter (3212) having a side opening (3214). During use of tubular cutter (3210), tether (3200) is threaded through side opening (3214), so that it is positioned to be cut by tubular cutter (3210).

While tubular cutter (3210) has a V-shaped cutting edge, any other appropriate notched feature may be used on a cutter, and other cutting edge configurations may also be used. As an example, FIG. 32B shows a tubular cutter (3220) having a curved cutting edge. Tubular cutter (3220) is externally disposed relative to a catheter (3222) having a side opening (3224). During use, a tether (3226) is threaded through side opening (3224), so that it can be severed by tubular cutter (3220). As another example, FIG. 32C shows a tubular cutter (3230) having an angled cutting edge. Tubular cutter (3230) is external to a catheter (3232) having a side opening (3234) through which a tether (3236) is threaded. As an additional example, FIG. 32D shows a tubular cutter (3240) having a serrated cutting edge. Tubular cutter (3240) is external to a catheter (3242) having a side opening (3244) through which a tether (3246) is threaded. Additional cutting edge configurations may be used, such as a saw-tooth cutting edge (not shown). The latter two variations may be useful, for example, when the tubular cutter is rotated or spun during the cutting process. In some variations, the perimeter of a side opening in a cutting catheter may be sharpened to help cut the tether. Tubular cutters, as well as other types of cutters, can be configured such that they operate either externally or internally to a catheter.

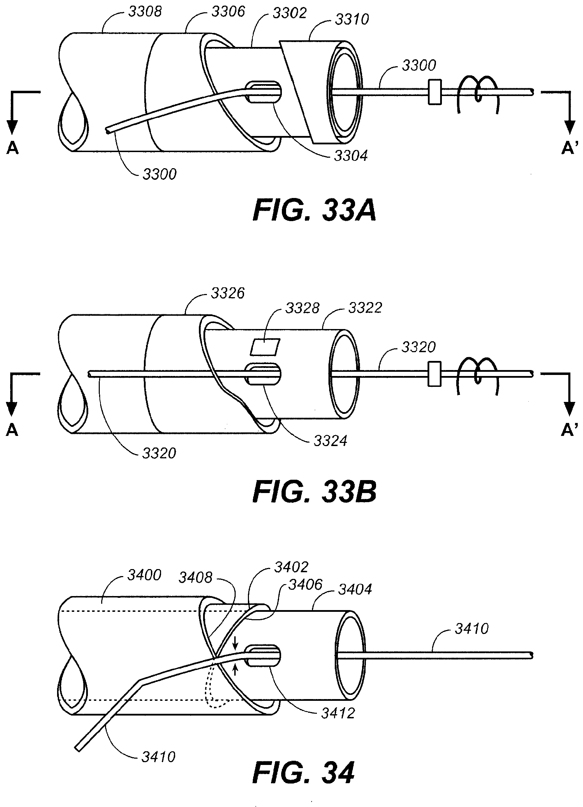

In some variations, tubular cutters can be used to sever a tether by cutting in a direction roughly perpendicular to the longitudinal axis of a catheter. For example, one concentric tube can be rotated relative to a second concentric tube to cut a tether. As an example, in FIG. 33A, a tether (3300) enters a catheter (3302) and exits through a side opening (3304) in the catheter. A tubular cutter (3306) is configured such that when it is rotated about the longitudinal axis A-A' of catheter (3302), it can slice tether (3300). For example, tubular cutter (3306) can have an angled shape such that when it rotates about longitudinal axis A-A', it cuts tether (3300). In some variations, tubular cutter (3306) is attached to a flexible tube (3308), as shown in FIG. 33A. In certain variations (also shown in FIG. 33A), a blocking structure (3310) is disposed on catheter (3302). Blocking structure (3310) can have any suitable shape, and can serve as a base against which tether (3300) can be pushed during the cutting process. Blocking structure (3310) can be attached to, part of, or integral with, catheter (3302).

Other variations can also be used. As an example, FIG. 33B shows a tether (3320) that is threaded through a catheter (3322) and that exits through a side opening (3324) in the catheter. A tubular cutter (3326) is disposed around catheter (3322). Tubular cutter (3326) has a cutting edge that is shaped to cut tether (3320) in a direction generally orthogonal to longitudinal axis A-A' of catheter (3322) as it is rotated around longitudinal axis A-A'. Optionally, a blocking structure (3328) can be provided on catheter (3322) such that tether (3320) is pushed against blocking structure (3328) during the cutting process. Blocking structure (3328) can be any suitable shape or have any suitable configuration and can be attached to, part of, or integral with, catheter (3322). While not shown, in some variations, tubular cutters such as those illustrated in FIGS. 33A and 33B can be configured such that they are internal to the catheter.

In some variations, a pair of concentric tubular cutters can be used to cut a tether. The concentric tubular cutters can be either internal or external to a catheter. For example, as illustrated in FIG. 34, two concentric tubular cutters (3400) and (3402) are externally disposed relative to a catheter (3404). Tubular cutters (3400) and (3402) can be rotated about the longitudinal axis A-A' of catheter (3404) in opposite directions (indicated by solid arrows). Thus, the cutting edges (3406) and (3408) of the tubular cutters can cut a tether (3410) that is routed through a side opening (3412) in catheter (3404) in a scissor- like fashion. Cutting edges (3406) and (3408) can be sharpened in such a way to enable cutting edges (3406) and (3408) to pass each other as closely as possible.

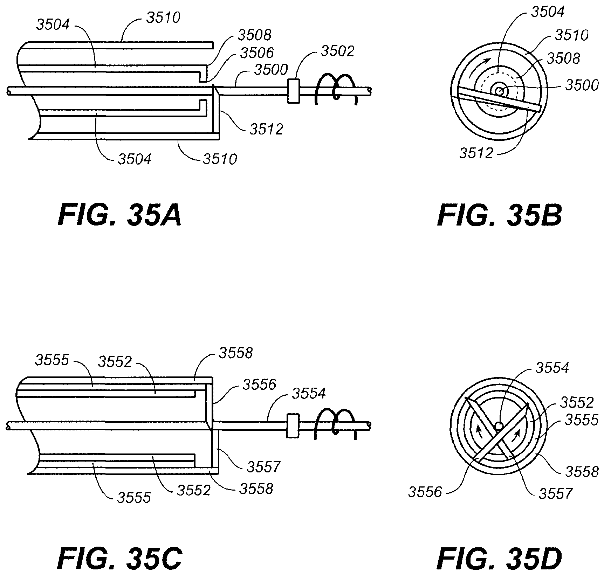

In some variations, a tether may not exit a catheter through a side opening in a catheter. In some such variations, a cutter can be mounted on a tube concentric to the catheter, either externally or internally, and rotated to cut the tether. For example, as shown in FIG. 35A, excess tether (3500) proximal to a locking element (3502) enters a catheter (3504) through its end opening (3506). Optionally, catheter (3504) can have a rim (3508) that restricts the diameter of its end opening (3506). A concentric tube (3510) has attached thereto a blade (3512), which can be rotated to sever excess tether (3500). FIG. 35B illustrates the operation of blade (3512) on tether (3500) as it is rotated.

Alternatively, and as shown in FIG. 35C, two concentric tubes (3555) and (3558) can be provided around a catheter (3552). Tube (3555) has a blade (3556) attached to its end, and tube (3558) has a blade (3557) attached to its end. Blades (3556) and (3557) are oriented generally perpendicular to the longitudinal axes of tubes (3555) and (3558). Tubes (3555) and (3558) are rotated in opposite directions about their respective longitudinal axes to cut a tether (3554). FIG. 35D illustrates the operation of blades (3556) and (3557) on tether (3554) as they are rotated. Blades (3556) and (3557) can be configured such that sharpened edges pass each other closely enough and at such angles to facilitate cutting.

Cutting blades as described herein can have any suitable configuration. For example, cutting blades may have cutting edges that are angled, V-shaped, curved, etc. Concentric tubes can be mounted either external or internal to a catheter. For example, one tube can be external while the other is internal.

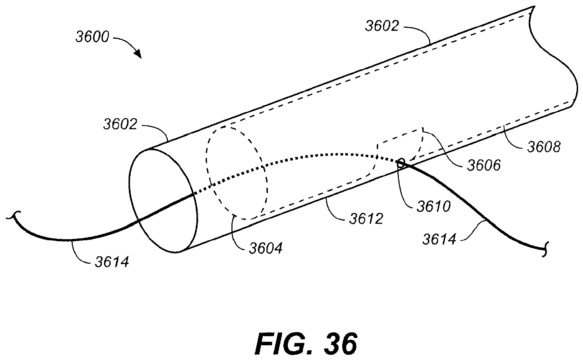

A further example of a device in the form of a tubular cutter configuration that may be used to cut one or more tethers is shown in FIG. 36. As shown in FIG. 36, a cutting device (3600) is formed of an outer tube (3602) and an inner tube (3604) concentrically disposed within outer tube (3602). Inner tube (3604) has a cut-out (3606) formed in its wall (3608), and outer tube (3602) has an opening (3610) formed in its wall (3612). In FIG. 36, inner tube (3604) is aligned within outer tube (3602) such that cut-out (3606) is in the location of opening (3610). A tether (3614) is threaded into both outer tube (3602) and inner tube (3604), and through cut-out (3606) and opening (3610). During use, inner tube (3604) is moved proximally and/or distally within outer tube (3602), and/or is rotated within outer tube (3602), thereby causing an edge of cut-out (3606) to contact and sever tether (3614). In some variations, cut-out (3606) can have one or more sharpened edges to facilitate cutting of a tether. As an example inner tube (3604) may be formed of a metal and may have sharpened metallic edges.

As described above, a tether cutter may comprise any appropriate structure or material. In addition to the tubular cutters described above, other examples of tether cutters include tether cutters that cut by heat, electricity, chemical reaction, or the like. For example, in some variations, a tether cutter may comprise an electrode or filament through which electrical energy is applied to cut a tether.

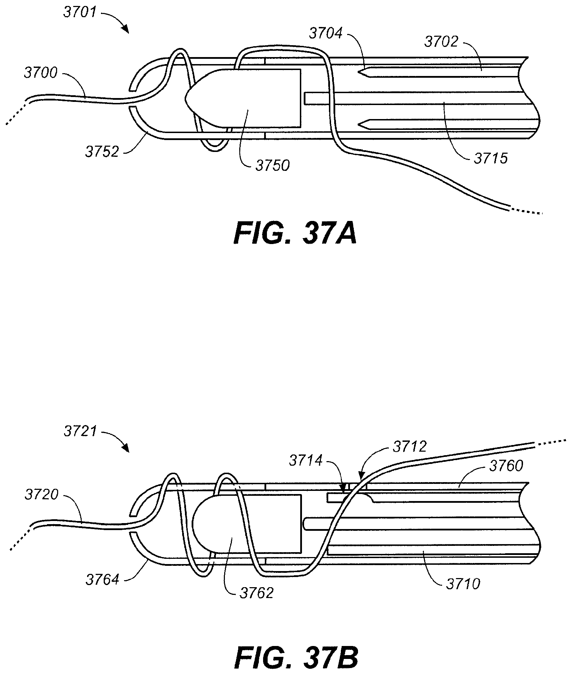

While locking devices and cutting devices have been described, in some variations, a single device can provide both locking and cutting functions. For example, FIGS. 37A and 37B illustrate different tether cutters that may be incorporated into a device including a detachable locking element.

FIG. 37A shows a device (3701) that is in the form of a catheter and that comprises a detachable locking element comprising a plug (3750) and a hollow portion (3752) configured to receive the plug. The device also includes a tubular tether cutter (3702) having a sharpened outer edge (3704), and a pushing member (3715) that passes through cutter (3702). Device (3701) further includes guides which can guide a tether (3700) through the device and position the tether for cutting. As shown in FIG. 37A, tether (3700) is positioned through the device so that it can be readily cut by cutter (3702) when the cutter is brought forward (e.g., moving the cutter distally). In FIG. 37A, cutter (3702) has at least one edge (e.g., over half of the cutter's circumference) so that at least one end of the tether (e.g., the end contacting the more proximal end of the tether) is cut by the cutter.

As described above, other types of tether cutters may be used as well. For example, FIG. 37B shows a combination locking and cutting device (3721) comprising a similar tubular tether cutter (3710) that is configured to cut the tether when the cutter is retracted proximally. In FIG. 37B, cutter (3710) has a passage (3712) through which a tether (3720) passes, and where at least a portion (3714) of the cutter is sharp. Tether (3720) also passes through the wall (3760) of the device (configured as a catheter in FIG. 37B, although other suitable configurations may be used). The end of the tether can be cut by drawing the tether taut after securing the locking element of the device and then moving the cutter against the tether so that it is cut. As shown in FIG. 37B, the locking element of device (3721) comprises a plug (3762) and a hollow portion (3764) configured to receive the plug.

Additional variations of devices that serve both a tether-locking function and a tether-cutting function may be used. For example, in certain variations, a device may comprise a tether cutter that is configured to cut a tether when the cutter is pulled proximally (e.g., like cutter (2504) in FIG. 25 above), as well as a locking element comprising an interlocking feature, such as interlocking feature (712) (shown in, and described with reference to, FIGS. 7A-7D). Other suitable combinations of locking and cutting elements may also be used, as appropriate.

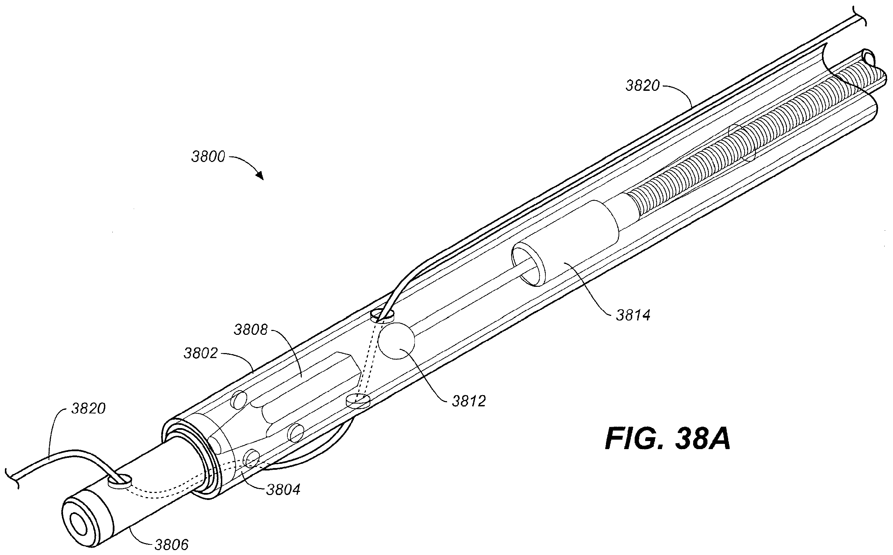

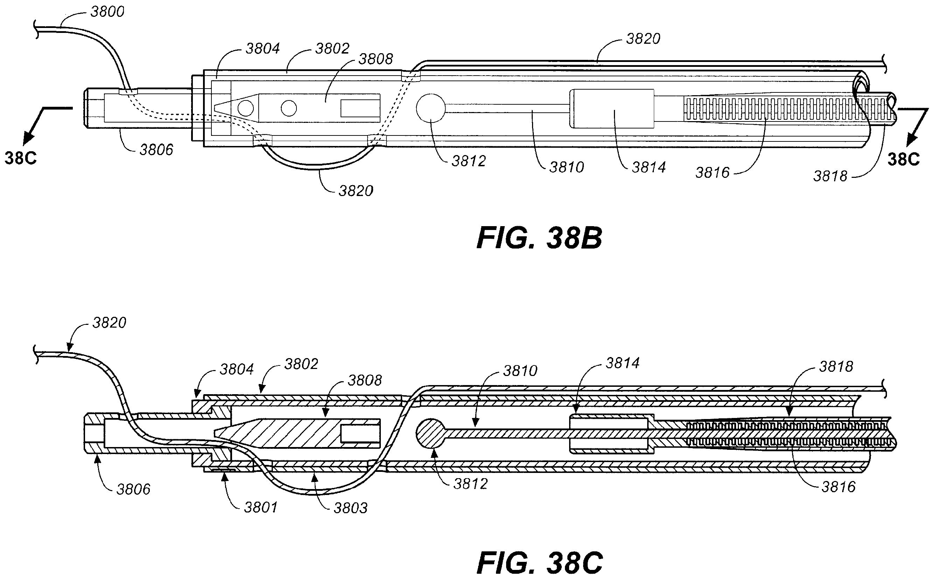

For example, FIGS. 38A-38C show a device that serves the combined functions of locking and cutting. As shown in FIGS. 38A-38C, a locking and cutting catheter (3800) includes an outer sheath (3802) surrounding a coupling tube (3804), which is coupled to a locking element (3806) (as shown, in the form of a locking tube, although other configurations may be used). Outer sheath (3802) includes a radiopaque marker band (3801), as well as a slot (3803) that allows the outer sheath to slide over coupling tube (3804) without resulting in any interference with tether routing. A plug (3808) is disposed within coupling tube (3804), and is configured to form an interference fit within locking element (3806). A wire (3810) having a guide (3812) at its distal end may be used to push plug (3808) into locking element (3806). Wire (3810) extends through a cutting element (3814), and then through a coil (3816) surrounded by a support tube (3818).

As shown in FIGS. 38A-38C, a tether (3820) is routed through various openings in the walls of sheath (3802), coupling tube (3804), and locking element (3806). When wire (3810) and guide (3812) are pushed in a distal direction, this causes plug (3808) to become pushed into locking element (3806), thereby securing the portion of tether (3820) disposed within locking element (3806). It also causes cutting element (3814) to sever tether (3820) at a location that is proximal to the location at which tether (3820) is secured or locked. Thus, device (3800) can serve the dual functions of both locking and cutting a tether. However, in some variations, wire (3810) may be slidable within coil (3816) which, in turn, can be fixedly attached to cutting element (3814). As a result, locking can be accomplish without advancement of the cutting element. The cutting element may thereafter be separately moved to effect cutting of the tether.

While certain variations of locking and cutting devices and methods have been described above, other variations may be used. As an example, in some variations, a cutting device may be used to cut a tether that is not under tension. In such variations, the tether may be cut, for example, by forcing the tether against a wall of the cutting device and using the wall as a backing for cutting the tether. Moreover, some variations of devices may be used to provide a cinching effect with a tether. These devices can be used for any surgery where these functions (or combinations thereof) are desired. Locking, cutting, and cinching devices are described, for example, in U.S. Patent Application Publication Nos. US 2006/0190030 A1, US 2006/0122633 A1, and US 2008/0172035 A1.

While the methods and devices have been described in some detail here by way of illustration and example, such illustration and example is for purposes of clarity of understanding only. It will be readily apparent to those of ordinary skill in the art in light of the teachings herein that certain changes and modifications may be made thereto without departing from the scope of the appended claims.

## Claims

1. A device for locking and/or cutting tethers used to tighten or compress tissue comprising:
an elongated member (2502) comprising a wall portion (2508) and a lumen (2503) defined by the wall portion, wherein the wall portion comprises an inner surface and an outer surface; and
a semitubular or tubular cutter (2504) disposed within the lumen and having a proximal end and a distal end, wherein the proximal end has a sharpened cutting edge (2506), wherein the wall portion comprises first and second openings (2510, 2512),
**characterized in that** the wall portion comprises a third opening, with the third opening distal from the second opening, the openings positioned such that a tether (2514), when extended at the same time through the first, second, and third openings, crosses the lumen between the first and second openings, and forms a path that is substantially parallel with the elongated member along the outer surface of the wall portion of the elongated member between the second and third openings, and wherein the cutter is located between the second and third openings and is movable toward a proximal end of the elongated member to cut a first portion of the tether without simultaneously cutting a second portion of the tether.

2. The device of claim 1, wherein the cutter (2504) is tubular.

3. The device of claim 1 or claim 2, wherein the first (2510) and second (2512) openings are positioned such that a tether (2514), when extended therethrough, forms a substantially diagonal path across the lumen (2503) of the elongated member (2502).

4. The device of any one of the preceding claims, wherein the elongated member (2502) comprises a catheter.

5. The device of any one of the preceding claims, further comprising a locking element (205) configured to secure a tether (2514).

6. The device of claim 5, wherein the elongated member (2502) has a proximal portion and a distal portion, and the locking element is releasably coupled to the distal portion of the elongated member (2502).

7. The device of claim 5, wherein the locking element comprises a plug and a locking tube configured to receive the plug.

8. The device of any one of the preceding claims, further comprising a guide, wherein the guide is configured to prevent the cutter (2504) from cutting the wall portion (2508).

9. The device of claim 8 wherein the guide at least partially surrounds the cutter (2504).

10. The device of claim 8 wherein the guide is coupled to the cutter (2504).

11. The device of claim 9 or claim 10, wherein the guide is configured to contact the wall portion (2508) when the wall portion (2508) is curved.

12. A device for locking and/or cutting tethers used to tighten or compress tissue comprising:
an elongated member (3102, 3802) comprising a wall portion and a lumen (3122) defined by the wall portion, wherein the wall portion comprises an inner surface and an outer surface; and
a semitubular or tubular cutter (3110, 3814) disposed within the lumen and having a proximal end and a distal end, wherein the distal end has a sharpened cutting edge, wherein the wall portion comprises first, second and third openings (3124, 3126), with the third opening distal from the second opening, the openings positioned such that a tether (3120, 3820), when extended at the same time through the first, second, and third openings, crosses the lumen between the first and second openings, and forms a path that is substantially parallel with the elongated member along the outer surface of the wall portion of the elongated member between the second and third openings, and wherein the cutter is located proximal to the first opening and is movable toward a distal end of the elongated member to cut a first portion of the tether without simultaneously cutting a second portion of the tether.

## Patentansprüche

1. Eine Vorrichtung zum Arretieren und/oder Abschneiden von Haltefäden, die zum Festziehen oder Komprimieren von Gewebe verwendet werden, die Folgendes beinhaltet:
ein längliches Element (2502), das einen Wandabschnitt (2508) und ein Lumen (2503), das durch den Wandabschnitt definiert wird, beinhaltet, wobei der Wandabschnitt eine innere Oberfläche und eine äußere Oberfläche beinhaltet; und
einen halbröhrenförmigen oder röhrenförmigen Abschneider (2504), der innerhalb des Lumens angeordnet ist und ein proximales Ende und ein distales Ende aufweist, wobei das proximale Ende eine geschliffene Schneidkante (2506) aufweist, wobei der Wandabschnitt eine erste und zweite Öffnung (2510, 2512) beinhaltet, **dadurch gekennzeichnet, dass** der Wandabschnitt eine dritte Öffnung beinhaltet, wobei die dritte Öffnung distal von der zweiten Öffnung liegt, wobei die Öffnungen so positioniert sind, dass ein Haltefaden (2514), wenn er sich zum gleichen Zeitpunkt durch die erste, zweite und dritte Öffnung erstreckt, das Lumen zwischen der ersten und zweiten Öffnung durchquert und eine Bahn bildet, die im Wesentlichen parallel zu dem länglichen Element entlang der äußeren Oberfläche des Wandabschnitts des länglichen Elements zwischen der zweiten und dritten Öffnung ist, und wobei der Abschneider sich zwischen der zweiten und dritten Öffnung befindet und zu einem proximalen Ende des länglichen Elements hin bewegt werden kann, um einen ersten Abschnitt des Haltefadens abzuschneiden, ohne gleichzeitig einen zweiten Abschnitt des Haltefadens abzuschneiden.

2. Vorrichtung nach Anspruch 1, wobei der Abschneider (2504) röhrenförmig ist.

3. Vorrichtung nach Anspruch 1 oder Anspruch 2, wobei die erste (2510) und zweite (2512) Öffnung so positioniert sind, dass ein Haltefaden (2514), wenn er sich durch diese hindurch erstreckt, eine im Wesentlichen diagonale Bahn über das Lumen (2503) des länglichen Elements (2502) hinweg bildet.

4. Vorrichtung nach einem der vorhergehenden Ansprüche, wobei das längliche Element (2502) einen Katheter beinhaltet.

5. Vorrichtung nach einem der vorhergehenden Ansprüche, die ferner ein Arretierungselement (205) beinhaltet, das zum Sichern eines Haltefadens (2514) ausgelegt ist.

6. Vorrichtung nach Anspruch 5, wobei das längliche Element (2502) einen proximalen Abschnitt und einen distalen Abschnitt aufweist und das Arretierungselement lösbar mit dem distalen Abschnitt des länglichen Elements (2502) gekoppelt ist.

7. Vorrichtung nach Anspruch 5, wobei das Arretierungselement einen Stopfen und ein Arretierungsrohr, das zum Aufnehmen des Stopfens ausgelegt ist, beinhaltet.

8. Vorrichtung nach einem der vorhergehenden Ansprüche, die ferner eine Führung beinhaltet, wobei die Führung dazu ausgelegt ist, zu verhindern, dass der Abschneider (2504) den Wandabschnitt (2508) abschneidet.

9. Vorrichtung nach Anspruch 8, wobei die Führung den Abschneider (2504) mindestens teilweise umgibt.

10. Vorrichtung nach Anspruch 8, wobei die Führung mit dem Abschneider (2504) gekoppelt ist.

11. Vorrichtung nach Anspruch 9 oder Anspruch 10, wobei die Führung dazu ausgelegt ist, den Wandabschnitt (2508) zu berühren, wenn der Wandabschnitt (2508) gekrümmt ist.

12. Eine Vorrichtung zum Arretieren und/oder Abschneiden von Haltefäden, die zum Festziehen oder Komprimieren von Gewebe verwendet werden, die Folgendes beinhaltet:
ein längliches Element (3102, 3802), das einen Wandabschnitt und ein Lumen (3122), das durch den Wandabschnitt definiert wird, beinhaltet, wobei der Wandabschnitt eine innere Oberfläche und eine äußere Oberfläche beinhaltet; und
einen halbröhrenförmigen oder röhrenförmigen Abschneider (3110, 3814), der innerhalb des Lumens angeordnet ist und ein proximales Ende und ein distales Ende aufweist, wobei das distale Ende eine geschliffene Schneidkante aufweist, wobei der Wandabschnitt eine erste, zweite und dritte Öffnung (3124, 3126) beinhaltet, wobei die dritte Öffnung distal von der zweiten Öffnung liegt, wobei die Öffnungen so positioniert sind, dass ein Haltefaden (3120, 3820), wenn er sich zum gleichen Zeitpunkt durch die erste, zweite und dritte Öffnung erstreckt, das Lumen zwischen der ersten und zweiten Öffnung durchquert und eine Bahn bildet, die im Wesentlichen parallel zu dem länglichen Element entlang der äußeren Oberfläche des Wandabschnitts des länglichen Elements zwischen der zweiten und dritten Öffnung ist, und wobei der Abschneider sich proximal zu der ersten Öffnung befindet und zu einem distalen Ende des länglichen Elements bewegt werden kann, um einen ersten Abschnitt des Haltefadens abzuschneiden, ohne gleichzeitig einen zweiten Abschnitt des Haltefadens abzuschneiden.

## Revendications

1. Dispositif de blocage et/ou de coupe d'attaches utilisées pour serrer ou comprimer un tissu comprenant :
un élément allongé (2502) comprenant une partie de paroi (2508) et une lumière (2503) définie par la partie de paroi, la partie de paroi comprenant une surface interne et une surface externe ; et
un outil de coupe semi-tubulaire ou tubulaire (2504) disposé à l'intérieur de la lumière et présentant une extrémité proximale et une extrémité distale, l'extrémité proximale présentant un bord de coupe aiguisé (2506), dans lequel la partie de paroi comprend des première et deuxième ouvertures (2510, 2512), **caractérisé en ce que** la partie de paroi comprend une troisième ouverture, la troisième ouverture étant distale à la seconde ouverture, les ouvertures étant positionnées de telle sorte qu'une attache (2514), quand elle est passée simultanément à travers les première, deuxième et troisième ouvertures, croise la lumière entre les première et deuxième ouvertures, et forme un chemin qui est sensiblement parallèle à l'élément allongé le long de la surface externe de la partie de paroi de l'élément allongé entre les deuxième et troisième ouvertures, et dans lequel l'outil de coupe est situé entre les deuxième et troisième ouvertures et est déplaçable vers une extrémité proximale de l'élément allongé pour couper une première partie de l'attache sans couper simultanément une seconde partie de l'attache.

2. Dispositif selon la revendication 1, dans lequel l'outil de coupe (2504) est tubulaire.

3. Dispositif selon la revendication 1 ou la revendication 2, dans lequel les première (2510) et deuxième (2512) ouvertures sont positionnées de telle sorte qu'une attache (2514), quand elle est passé à travers elles, forme un chemin sensiblement diagonal en travers de la lumière (2503) de l'élément allongé (2502).

4. Dispositif selon l'une quelconque des revendications précédentes, dans lequel l'élément allongé (2502) comprend un cathéter.

5. Dispositif selon l'une quelconque des revendications précédentes, comprenant en outre un élément de blocage (205) configuré pour fixer une attache (2514).

6. Dispositif selon la revendication 5, dans lequel l'élément allongé (2502) présente une partie proximale et une partie distale, et l'élément de blocage est couplé de manière détachable à la partie distale de l'élément allongé (2502).

7. Dispositif selon la revendication 5, dans lequel l'élément de blocage comprend un obturateur et un tube de blocage configuré pour recevoir l'obturateur.

8. Dispositif selon l'une quelconque des revendications précédentes, comprenant en outre un guide, dans lequel le guide est configuré pour empêcher l'outil de coupe (2504) de couper la partie de paroi (2508).

9. Dispositif selon la revendication 8, dans lequel le guide entoure au moins partiellement l'outil de coupe (2504) .

10. Dispositif selon la revendication 8, dans lequel le guide est couplé à l'outil de coupe (2504).

11. Dispositif selon la revendication 9 ou la revendication 10, dans lequel le guide est configuré pour faire contact avec la partie de paroi (2508) quand la partie de paroi (2508) est incurvée.

12. Dispositif de blocage et/ou de coupe d'attaches utilisées pour serrer ou comprimer un tissu comprenant :
un élément allongé (3102, 3802) comprenant une partie de paroi et une lumière (3122) définie par la partie de paroi, la partie de paroi comprenant une surface interne et une surface externe ; et
un outil de coupe semi-tubulaire ou tubulaire (3110, 3814) disposé à l'intérieur de la lumière et présentant une extrémité proximale et une extrémité distale, l'extrémité distale présentant un bord de coupe aiguisé, dans lequel la partie de paroi comprend des première, deuxième et troisième ouvertures (3124, 3126), la troisième ouverture étant distale à la deuxième ouverture, les ouvertures étant positionnées de telle sorte qu'une attache (3120, 3820), quand elle est passée simultanément à travers les première, deuxième et troisième ouvertures, croise la lumière entre les première et deuxième ouvertures, et forme un chemin qui est sensiblement parallèle à l'élément allongé le long de la surface externe de la partie de paroi de l'élément allongé entre les deuxième et troisième ouvertures, et dans lequel l'outil de coupe est situé à une position proximale à la première ouverture et est déplaçable vers une extrémité distale de l'élément allongé pour couper une première partie de l'attache sans couper simultanément une seconde partie de l'attache.
